(19) **Europäisches Patentamt**
**European Patent Office**
**Office européen des brevets**

(11) **EP 1 231 271 B2**

(12) **NEW EUROPEAN PATENT SPECIFICATION**
After opposition procedure

(45) Date of publication and mention
of the opposition decision:
**15.06.2011 Bulletin 2011/24**

(45) Mention of the grant of the patent:
**29.12.2004 Bulletin 2004/53**

(51) Int Cl.:
**C12N 15/48** (2006.01)      **C12Q 1/68** (2006.01)
**C12Q 1/70** (2006.01)

(21) Application number: **02008713.6**

(22) Date of filing: **18.10.1985**

(54) **DNA fragments of the GAG gene of LAV**

DNS-Fragmente des GAG-Gens von LAV

Fragments d'ADN du gène GAG de LAV

(84) Designated Contracting States:
**AT BE CH DE FR GB IT LI LU NL SE**

(30) Priority: **18.10.1984 FR 8416013**
        **16.11.1984 GB 8429099**
        **21.01.1985 GB 8501473**

(43) Date of publication of application:
**14.08.2002 Bulletin 2002/33**

(60) Divisional application:
**04077731.0 / 1 568 767**

(62) Document number(s) of the earlier application(s) in
accordance with Art. 76 EPC:
**01111330.5**
**91113062.3 / 0 462 627**
**90105190.4 / 0 387 915**
**85905513.9 / 0 201 540**

(73) Proprietors:
• **INSTITUT PASTEUR**
  **75724 Paris Cedex 15 (FR)**
• **CENTRE NATIONAL DE LA RECHERCHE**
  **SCIENTIFIQUE**
  **75704 Paris Cedex 16 (FR)**

(72) Inventors:
• **Montagnier, Luc**
  **92350 Le Plessis-Robinson (FR)**
• **Krust, Bernard**
  **75012 Paris (FR)**
• **Chamaret, Solange**
  **75015 Paris (FR)**
• **Clavel, Francois**
  **75002 Paris (FR)**
• **Chermann, Jean-Claude**
  **13260 Cassis (FR)**
• **Barre-Sinoussi, Francoise**
  **92310 Issy-Les-Moulineaux (FR)**
• **Alizon, Marc**
  **75005 Paris (FR)**
• **Sonigo, Pierre**
  **75015 Paris (FR)**
• **Stewart, Cole**
  **92320 Chatillon (FR)**
• **Danos, Olivier**
  **75015 Paris (FR)**
• **Wain-Hobson, Simon**
  **78180 Montigny-Le-Bretonneux (FR)**

(74) Representative: **Desaix, Anne**
**Ernest Gutmann - Yves Plasseraud S.A.S.**
**3, rue Auber**
**75009 Paris (FR)**

(56) References cited:
**EP-A- 0 178 978**

• **DATABASE MEDLINE [Online] US NATIONAL LIBRARY OF MEDICINE (NLM), BETHESDA, MD, US; 1984 MONTAGNIER ET A.: "Lymphadenopathy-associated virus and its etiological role in AIDS" XP002208978 & PRINCESS TAKAMATSU SYMPOSIUM, vol. 15, 1984, pages 319-331,**
• **ALIZON ET AL.: "Molecular cloning of lymphadenopathy-associated virus" NATURE, vol. 312, 1984, pages 757-760, XP002208977**

EP 1 231 271 B2

**Description**

The present invention relates to DNA fragments of the gag gene, of the virus of lymphadenopathies (denoted below by the abbreviation LAS) and of the acquired immuno-depressive syndrome (denoted below by the abbreviation AIDS).

The causative agent of LAS or AIDS, a retrovirus, has been identified by F. BARRE-SINOUSSI et al, Science, 220, 868 (1983). It has the following characteristics. It is T-lymphotropic; its prefered target is constituted by Leu 3 cells (or T4 lymphocytes) ; it has reverse transcriptase activity necessitating the presence of Mg + and exhibits strong affinity for poly(adenylate-oligodeoxy-thymidylate)(poly(A)-oligo(dT)12-18). It has a density of 1.16-1.17 in a sucrose gradient, an average diameter of 139 nanometers; and a nucleus having an average diameter of 41 nanometers. Antigens of said virus, particularly a protein p25 are recognised immunologically by antibodies contained in serums taken up from patients afflicted with LAS or AIDS. The p25 protein, which is a core protein, is not recognised immunologically by the p24 protein of the HTLVI and II viruses. The virus is also free of a p19 protein which is immunologically cross-reactive with the p19 proteins of HTLVI and HTLVII.

Retroviruses of this type (sometimes denoted by the generic abbreviation LAV) have been filed in the National Collection of Micro-organism Cultures of the INSTITUT PASTEUR of Paris, under numbers I-232, I-240 and I-241. Virus strains similar to LAV in all respects from the morphological and immunological point of view have been isolated in other laboratories. Reference is made by way of examples to the retrovirus strains named HTLV-III isolated by R.C. GALLO et al., Science, 224, 500 (1984) and by M.G. SARNGADHARAN et al., Science 224, 506 (1984) respectively and to the retrovirus isolated by M. JAY LEVY et al., Science, 225, 840-842 (1984), which virus was designated ARV. For the ease of language the last mentioned viruses, as well as others which have equivalent morphological and immunological properties, will be designated hereafter under the generic designation "LAV". Reference is also made to European patent application filed 14 September 1984, with the priority of British patent application number 83 24800 filed 15 september 1983 as regards a more detailed description of the LAV retroviruses or the like and of the uses to which extracts of these viruses give rise.

Initially the core antigens were the main antigens of the virus lysates or extracts which were recognised by serums of patients infected with AIDS or LAS, in the test systems which had then been used. A p42 protein, presented as consisting of an envelope protein, had been detected too. In the same manner GALLO et al disclosed a p41 protein which was also deemed to be on a possible component of the viru envelope.

Processes for obtaining a LAV virus have also been described. Reference may be made particularly to the article already mentioned of F. BARRE-SINOUSSI et al., as regards the preparation of the virus in T lymphocyte cultures derived either from blood, or from the umbilical cord, or also from bone marrow cells of adult donors in good health. This process comprises particularly the following essential steps :

-    producing a viral infection of these T lymphocytes, after activation by a lectin mitogen, with a viral suspension derived from a crude supernatant liquor of lymphocytes producing the virus (initially obtained from a patient infected with AIDS or LAS),
-    culturing cells infected with TCGF, in the presence of anti-$\alpha$-interferon sheep serum,
-    effecting purification of the virus produced (production starts generally between the 9th and the 15th day following infection and lasts from 10 to 15 days), which purification comprises precipitating the virus in polyethylenglycol in order to produce a first concentration of the virus, then centrifugating the preparation obtained in a 20-60 % sucrose gradient or in an isotonic gradient of metrizanide (sold under the trade mark NYCODENZ by NYEGAARD, Oslo) and recovering the virus with the band having a density of 1.16-1.17 in the sucrose gradient or of 1.10-1.11 in the NYCODENZ®gradient.

The LAV virus may also be produced from permanent cell lines of type T, such as the CEM line, or from B lymphoblastoid cell lines, such as obtained by the transformation of the lymphocytes derived from a healthy donor with the Epstein-Barr virus, for instance as disclosed in French patent application Nr. 84 07151 filed May 9, 1984. The permanent cell lines obtained produce continuously a virus (designated as LAV-B in the case of the B lymphoblastoid cell lines) which possesses the essential antigenic and morphological features of the LAV viruses (except that it is collected in a density band sometimes slightly higher than in the preceding case (particularly 1.18) in sucrose. The final purification of the virus can also be carried out in a NYCODENZ®gradient.

.Reference is made to the article of Schupbach et al, Science, vol. 224, pages 503-505. 4 May 1984. This article discloses a work with respect to different antigens of the HTLV-III retrovirus.

Results are presented including a reference to an antigen having migrated on an electrophoresis gel at a migration distance corresponding to a molecular weight of approximately 110,000.

Schupbach et al mentioned that this antigen was detected in a virus preparation but was below limit of detection in the cells.

EP-A-0 178 978 discloses clone λJ19 of LAV and the sequence of parts of LAV genome.

DATABASE MEDLINE US NATIONAL LIBRARY OF MEDICINE, BETHESDA, MD, US; 1984 MONTAGNIER ET AL discloses that LAV is a novel human retrovirus which genome is 9.2 kb long.

A method for cloning DNA sequences hybridizable with the genomic RNA of LAS has already been disclosed in British Patent Application Nr. 84 23659 filed on September 19, 1984. Reference is hereafter made to that application as concerns subject matter in common with the further improvements to the invention disclosed herein.

The application discloses purified unaltered virus forms (or viruses less altered by the purification procedures resorted to) and processes for obtaining said. unaltered purified viruses.

The present invention further aims at providing additional new means which should not only also be useful for the detection of LAV or related viruses (hereafter more generally referred to as "LAV viruses"), but also have more versatility, particularly in detecting specific parts of the genomic DNA of said viruses whose expression products are not always directly detectable by immunological methods. The present invention further aims at providing polypeptides containing sequences in common with polypeptides comprising antigenic determinants included in the proteins encoded and expressed by the LAV genome occuring in nature. Finally the invention also discloses immunogenic polypeptides, and more particularly protective polypeptides for use in the preparation of vaccine compositions against AIDS or related syndroms.

The present invention relates to additional DNA fragments, hybridizable with the genomic RNA of LAV as they will be disclosed hereafter, as well as with additional cDNA variants corresponding to the whole genomes of LAV viruses. It further relates to DNA recombinants containing said DNAs or cDNA fragments.

An unaltered purified LAV retrovirus distinguishes from those which have been defined above, in that it includes an amount of one or several envelope antigens, sufficient to be visualized when the virus is labelled with $^{35}$S-cystein, free of unlabelled cystein in a proportion of 200 microcuries per ml of medium. these antigens, among which particularly glycoproteins, are recognised selectively in vitro by serums of patients affected with SIDA or SLAs or by the serums of asymptomatic carriers of the virus.

The invention discloses also the antigens themselves, which possess also the capability of being recognised by serums of patients infected with AIDS or LAS or by serums of persons who have been exposed to LAV viruses or those analogous with the latter. These antigens have also the characteristic of forming complexes with concanavaline A, said complex being dissociatable in the presence of O-methyl-α-D-mannopyranoside. The antigens according to the invention can also bind to other lectins for example those known under the name "LENTYL-LECTIN".

Preferred antigens disclosed are constituted by proteins or glycoproteins.

The invention discloses also the process for producing the viruses according to the invention. This process distinguishes essentially from those recalled above at the level of the final purification operation. In particular, the purification step of the process according to the invention is no longer carried out in gradients, but involves the performance of differential centrifugations effected directly on the supernatants of the culture media of the producing cells. These centrifugation operations comprise particularly a first centrifugation at an angular centrifugation velocity, particularly of 10,000 rpm, enabling the removal of non-viral constituents, more particularly of cellular constituents, then a second centrifugation at higher angular velocity, particularly at 45,000 rpm, to obtain the precipitation of the virus itself. In preferred embodiments, the first centrifugation at 10,000 rpm, is maintained for 10 minutes and the second at 45,000 rpm, for 20 minutes. These are, of course, only indicative values, it being understood that it remains within the ability of the specialist to modify the centrifugation conditions, to provide for the separation of the cellular constituents and of the viral constituents.

This modification of the purification process results in the production of viral preparations from which the antigen mentioned can then be isolated more easily, than from virus preparations purified by the previous methods. In any event, the viruses finally obtained by the process of the present invention are more easily recognised by serums of patients or of persons who have been exposed to the LAV virus or to morphologically and antigenically similar strains.

The antigens disclosed can themselve be obtained from the above disclosed viruses, by lysis (or other suitable processing) of the latter in the presence of any suitable detergent and by recovery and separation of the antigens released. Advantageously, the lysis of the virus is effected in the presence of aprotinin or of any other agent suitable for inhibiting the action of proteases. The separation of the antigens according to the invention can then be carried out by any method known in itself ; for example, it is possible to proceed with a separation of the proteins by employing their respectively different migrations in a predetermined gel, the protein sought being then isolated from the zone of the gel in which it would normally be found in an electrophoresis operation under well determined conditions, having regard to its molecular weight. The antigens can however be separated from the lysate of the abovesaid viruses, due to their affinity for lectins, in particular concanavaline A or lentyl-lectin. The lectin used is preferably immobilised on a solid support, such as the cross linked polymer derived from agarose and marketed under the trade mark SEPHAROSE. After washing of the fixed antigens with a suitable buffer, the antigens can be eluted in any suitable manner, particularly by resorting to a O-methyl-α-D-mannopyranoside in solution.

A more thorough purification of these antigens can be performed by immunoprecipitation with the serums of patients known to possess antibodies effective against said protein, with concentrated antibody preparations (polyclonal antibodies) or again with monoclonal antibodies, more particularly directed against the antigen according to the invention, in particular that having the molecular weight of 110,000, denoted below by the abbreviation gp110.

Additional characteristics of the invention will appear also in the course of the description which follows of the isolation of a virus according to the invention and of antigens. Reference will be made to the drawings in which :

Figure 1 is derived from a photographic reproduction of gel strips which have been used to carry out electrophoreses of lysate extracts of T lymphocytes, respectively infected and uninfected (controls) by a LAV suspension.

Figure 2 is the restriction map of a complete LAV genome (clone λJ19).

Figures 3a to 3e are the complete sequence of a LAV viral genome.

Figures 4 and 5 show diagrammatically parts of the three possible reading phases of LAV genomic RNA, including the open reading frames (ORF) apparent in each of said reading phases.

Figure 6 is a schematic representation of the LAV long terminal repeat (LTR).

## I - PRODUCTION OF THE VIRUS AND OF ANTIGENS

T lymphocytes derived from a healthy donor and infected with LAV1, under the conditions described by F. BARRE-SINOUSSI et Coll., on CEM cells derived from a patient afflicted with leukemia and also infected in vitro with LAV1, were kept under cultivation in a medium containing 200 microcuries of $^{35}$S-cystein and devoid of unlabelled cystein. The infected lymphocytes were cultured in a non denaturating medium to prevent the degradation of the antigen sought. The supernatant liquor from the culture medium was then subjected to a first centrifugation at 10,000 rpm for 10 minutes to remove the non viral components, then to a second centrifugation at 45,000 rpm for 20 minutes for sedimenting the virus. the virus pellet was then lysed by detergent in the presence of aprotinin (5 %) particularly under the conditions described in the article of F. BARRE-SINOUSSI et Coll.

The same operation was repeated on lymphocytes taken up from a healthy donor as control.

The various lysates were then immuno-precipitated by serums of patients infected with AIDS or with LAS. Serums originating from healthy donors or of donors infected with other diseases were immunoprecipitated too. The media were then subjected to electrophoreses in a SDS-polyacrylamide gel.

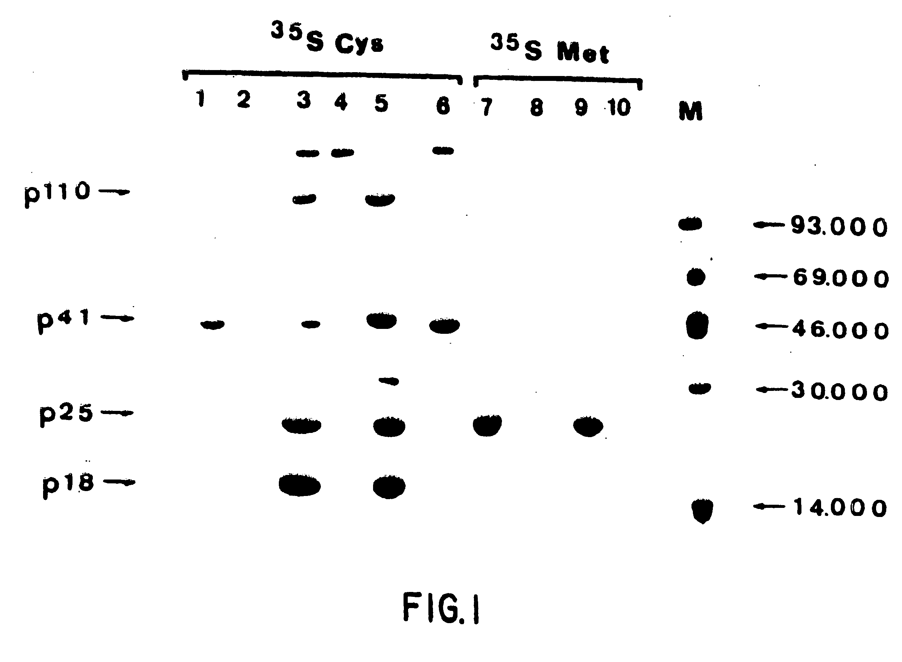

The results are indicated in figure 1. The gel strips numbered from 1 to 6 were obtained from preparations labelled by $^{35}$S-cystein. The strips numbered 7 to 10 show results observed on infected or uninfected lymphocyte preparations labelled with $^{35}$S-methionine. Finally the strip M corresponds to the migration distances of the standard proteins identified above, whose molecular weights are recalled in the right hand portion of the figure.

The references to the labelled viral proteins appear on the left handside of the figure.

It is noted that columns 7 and 9 show the specific protein p25 of LAV, labelled with $^{35}$S-methionin. The same protein is absent on strips 8 and 10 corresponding to results obtained with a preparation originating from healthy lymphocytes.

Columns 3 and 5 correspond to the results which have been observed on preparations obtained from lymphocytes infected and labelled with $^{35}$S-cystein. The proteins p25 and p18, the characteristic core proteins of LAV, and the glycoprotein gp110, also specific of LAV, were also present. Images corresponding to a protein p41 (molecular weight of the order of 41,000) appeared in the various preparations, although less distinctly.

The virus according to the invention and the antigen according to the invention can be either precipitated by lectins, particularly concanavaline A, or fixed to a SEPHAROSE-concanavaline A column. Particularly the purification of the envelope glycoproteins can be carried out as follows. This fixation can particularly be carried out by contacting a lysate of the LAV virus dissolved in a suitable buffer with concanavaline-A bound to SEPHAROSE®. A suitable buffer has the following composition :

| Tris | 10 mM |
|---|---|
| NaCl | 0.15 M |
| CaCl$_2$ | 1 mM |
| MgCl$_2$ | 1 mM |

Detergent marketed under the trade mark TRITON 1 %

| pH | 7.4 |
|---|---|

When the fixation has been achieved, the SEPHAROSE® concanavaline A is washed with a buffer of the same composition, except that the TRITON® concentration is lowered to 0.1 %. The elution is then effected with an 0.2 M O-

methyl-$\alpha$-D-mannopyranoside solution in the washing buffer.

The protein may be further concentrated by immuno-precipitation with antibodies contained in the serums of patients infected with AIDS or with polyclonal antibodies obtained from a serum derived from an animal previously immunised against the "unaltered" virus according to the invention or the abovesaid glycoprotein. The protein can then be recovered by dissociation of the complex by a solution having an adequate content of ionic salt. Preferably the antibody preparation is itself immobilised in a manner known in itself on an insoluble support, for instance of the SEPHAROSE® B type.

It is also possible to resort to monoclonal antibodies secreted by hybridomas previously prepared against gp 110. These monoclonal antibodies, as well as the hybridomas which produce them, also form part of the invention.

A technique for producing and selecting monoclonal antibodies directed against the gp110 glycoprotein is described below.

Immunisation of the mice

Groups of Balb/c mice from 6 to 8 weeks old mice were used. One group receives the virus carrying the abovesaid glycoprotein, another a purified glycoprotein gp110. The immunisation procedure, identical for all mice, comprises injecting 10 mg of the antigenic preparation in the presence of Freund complete adjuvant at day 0, then again but in the presence of Freund incomplete adjuvant at day 14 and without adjuvant at days 28 and 42. The three first injections are made intraperitoneally, the fourth intravenously.

Fusion and culture of the hybrids

The non secreting myeloma variant 5.53 P3 x 63 Ag8, resistant to azaguanine, itself derived from the MOPC-21 cell-line, is used. Fusion with immunised mouse splenocytes is carried out in the presence of polyethylene-glycol 4000 by the technique of FAZEKAS de st-GROTH and SCHEIDEGGER on the 45th day. The selection of the hybrids in RPMI 16-40 "HAT" medium is carried out in plates having 24 cups (known under the designation COSTAR) by resorting to the same culture techniques.

The hybridomas producing antibodies of adequate specificity are then cloned in plates having 96 cups, in the presence of a "feeder" layer of syngenic thymocytes. The producing clones thus selected are then expanded in 24 cup plates, still in the presence of thymocytes. When the confluence appears in one of the cups, the clone is injected intraperitoneally into a balb/c mouse which had received an injection of PRISTANE 8 days previously and/or kept in liquid culture.

Demonstration of the anti-LAV antibodies

Five different techniques enable characterisation of the clones producing antibodies of suitable specificity. In a first stage, the hybrids producing antibodies are determined by an ELISA test revealing mouse immunoglobulins in the supernatant liquors. From this first selection, supernatants are sought which have antibodies directed against viral constituents by means of an ELISA test revealing anti-LAV antibodies, or by immunofluorescence on the virus producing human cells. Finally the supernatant liquors are analysed by radioimmunoprecipitation of virus labelled with cystein and by the Western-Blot technique on viral preparation which permit the determination of the specificities of these anti-LAV antibodies.

RESULTS

Cells obtained from the various fusions are placed under culture in 648 cups. Their nicroscopic examination shows that the majority of these cups contain a single hybrid clone capable of growing in a "HAT" selective medium. More than 50 % among them produce antibodies giving rise to a positive response under ELISA antivirus examination. The most representative fusions are tested by the Western-Blot technique and several of them are subcloned, taking into account their respective specificities reactivities in antivirus ELISA and their behaviours under the culturing conditions. Those hybrids which are more particularly selected are those which produce antibodies which selectively recognise the viral glycoprotein gp110 having a molecular weight of about 110 KD. All the sub clonings give rise to clones producing antibodies which, after expression, are injected into syngenic mice. Analysis of the specificities of the antibodies present in the different ascites liquids confirms the specificity of the antibodies of said ascites with respect to gp110.

The monoclonal antibodies obtained can themselves be employed to purify proteins containing an antigenic site also contained in gp110. The invention relates therefore also to these processes of purification as such. This process is advantageously applied to virus lysates or T lymphocyte lysates or other cells producing LAV or the like, when care has been taken to avoid the uncontrolled separation of gp110 during the purification procedure of the virus, prior to lysis thereof. Needless to say that the process can also be applied to any solution containing gp110 or a protein, polypeptide or glycoprotein comprising an antigenic site normally carried by the envelope protein and recognised by the monoclonal antibody. For practising this process, the monoclonal antibodies are advantageously immobilised on a solid support, preferably adapted to affinity chromatography operations. For example, these monoclonal antibodies are fixed to an agarose lattice with three-dimensional cross-linking, marketed under the trade mark SEPHAROSE by the Swedish company PHARMACIA A.G., for example by the cyanogen bromide method.

The invention therefore also relates to a process for separating the antigens concerned, which process comprises contacting a mixture of antigens, including those of interest (for instance a virus lysate or extract), with an affinity column bearing the abovesaid monoclonal antibodies, to selectively fix polypeptides, proteins or glycoproteins selectively recognized by said monoclonal antibodies, recovering the latter by dissociation of the antigen-antibody complex by means of a suitable buffer, particularly a solution of adequate ionic strength, for example of a salt, preferably ammonium acetate (which leaves no residue upon freeze drying of the preparation)or a solution acidified to a pH 2-4 or to a glycine buffer at the same pH and recovering the eluted polypeptides, proteins or glycoproteins.

It is self-evident that the invention relates also to polypeptide fragments having lower molecular weights and carrying antigenic sites recognizable by the same monoclonal antibodies. Fragments of smaller sizes may be obtained by resorting to known techniques. For instance such a method comprises cleaving the original larger polypeptide by enzymes capable of cleaving it at specific sites. By way of examples of such proteins, may be mentioned the enzyme of Staphylococcyus aureus V8, $\alpha$-chymotrypsine, "mouse sub-maxillary gland protease" marketed by the BOEHRINGER company, Vibrio alginolyticus chemovar iophagus collagenase, which specifically recognises said pcptides Gly-Pro and Gly-Ala, etc..

Particularly the invention relates more particularly to polypeptides encoded by cDNA fragments defined hereafter. It also relates to the nucleic acid fragments themselves, including a cDNA variant corresponding to a whole LAV retroviral genome, characterized by a series of restriction sites in the order hereafter (from the 5' end to the 3' end).

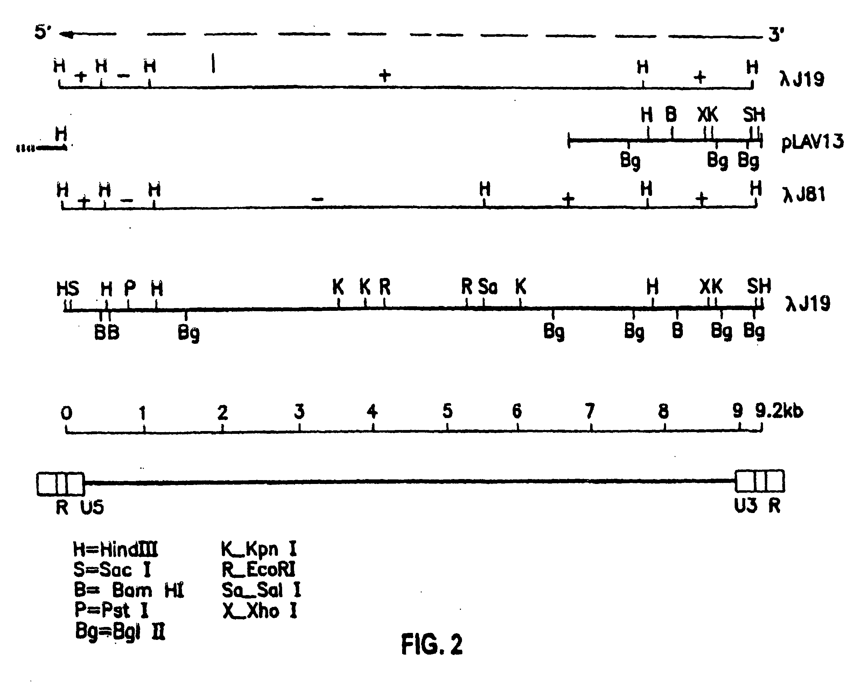

The coordinates of the successive sites of the whole LAV genome (see also restriction map of λJ19 in fig. 2) are indicated hereafter too, with respect to the Hind III site (selected as of coordinate 1) which is located in the R region. The coordinates are estimated with an accuracy of $\pm$ 200 bp :

| Hind III | 0 |
|---|---|
| Sac I | 50 |
| Hind III | 520 |
| Pst I | 800 |
| Hind III | 1 100 |
| Bgl II | 1 500 |
| Kpn I | 3 500 |
| Kpn I | 3 900 |
| Eco RI | 4 100 |
| Eco RI | 5 300 |
| Sal I | 5 500 |
| Kpn I | 6 100 |
| Bgl II | 6 500 |
| Bgl II | 7 600 |
| Hind III | 7 850 |
| Bam HI | 8 150 |
| Xho I | 8 600 |
| Kpn I | 8 700 |
| Bgl II | 8 750 |
| Bgl II | 9 150 |
| Sac I | 9 200 |
| Hind III | 9 250 |

Another DNA variant according to this invention optionally contains an additional Hind III approximately at the 5 550 coordinate.

Reference is further made to fig. 1 which shows a more detailed restriction map of said whole-DNA (λJ19).

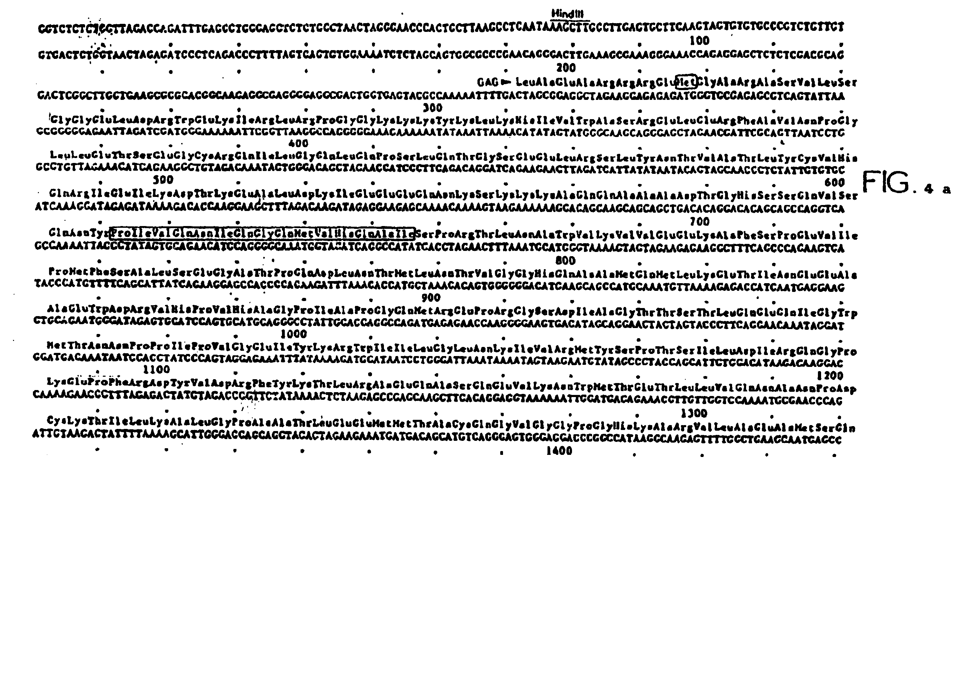

An even more detailed nucleotidic sequence of a preferred DNA according to the invention is shown in figs. 4a-4e hereafter.

The invention further relates to other preferred DNA fragments and polypeptide sequences (glycosylated or not glycosylated) which will be referred to hereafter.

SEQUENCING OF LAV

The sequencing and determination of sites of particular interest were carried out on a phage recombinant corresponding to λJ19 disclosed in the abovesaid British Patent application Nr. 84 23659. A nethod for preparing it is disclosed in that application.

The whole recombinant phage DNA of clone AJ19 (disclosed in the earlier application) was sonicated according to the protocol of DEININGER (1983), Analytical Biochem. 129, 216. The DNA was repaired by a Klenow reaction for 12 hours at 16°C. The DNA was electrophoresed through 0.8 % agarose gel and DNA in the size range of 300-600 bp was cut out and electroeluted and precipitated. Resuspended DNA (in 10 mM Tris, pH 8 ; 0,1 mM EDTA) was ligated into M13mp8 RF DNA (cut by the restriction enzyme SmaI and subsequently alkaline phosphated), using T4 DNA- and RNA-ligases (Maniatis T et al (1982) - Molecular cloning - Cold Spring Harbor Laboratory). An E. coli strain designated as TG1 was used for further study. This strain has the following genotype : Δlac pro, supE, thi.F'traD36, proAB, lacIq, ZΔM15, r⁻

This E. coli TGI strain has the peculiarity of enabling recombinants to be recognized easily. The blue colour of the cells transfected with plasmids which did not recombine with a fragment of LAV DNA is not modified. To the contrary cells transfected by a recombinant plasmid containing a LAV DNA fragment yield white colonies. The technique which was used is disclosed in Gene (1983), 26, 101.

This strain was transformed with the ligation mix using the Hanahan method (Hanahan D (1983) J. Mol. Biol. 166, 557). Cells were plated out on tryptone-agarose plate with IPTG and X-gal in soft agarose. White plaques were either picked and screened or screened directly in situ using nitrocellulose filters. Their DNAs were hybridized with nick-translated DNA inserts of pUC18 Hind III subclones of λJ19. This permitted the isolation of the plasmids or subclones of λ which are identified in the table hereafter. In relation to this table it should also be noted that the designation of each plasmid is followed by the deposition number of a cell culture of E. coli TGI containing the corresponding plasmid at the "Collection Nationale des Cultures de Micro-organismes" (C.N.C.M.) of the Pasteur Institute in Paris, France. A non-transformed TGI cell line was also deposited at the C.N.C.M. under Nr. I-364. All these deposits took place on November 15, 1984. The sizes of the corresponding inserts derived from the LAV genome have also been indicated.

TABLE

| Essential features of the recombinant plasmids | |
|---|---|
| - pJ19 - 1 plasmid (I-365)<br>    Hind III - Sac I - Hind III | 0.5 kb |
| - pJ19 - 17 plasmid (I-368)<br>    Hind III - Pst 1 - Hind III | 0.6 kb |
| - pJ19 - 6 plasmid (I-366)<br>    Hind III (5')<br>    Bam HI<br>    Xho I<br>    Kpn I<br>    Bgl II<br>    Sac I (3')<br>    Hind III | 1.5 kb |
| - pJ19-13 plasmid (I-367)<br>    Hind III (5')<br>    Bgl II<br>    Kpn I<br>    Kpn I<br>    Eco RI<br>    Eco RI<br>    Sal I<br>    Kpn I<br>    Bgl II | 6.7 kb |

(continued)

| Essential features of the recombinant plasmids | |
| --- | --- |
| Bgl II | |
| Hind III (3') | |

Positively hybridizing M13 phage plates were grown up for 5 hours and the single-stranded DNAs were extracted.

M13mp8 subclones of λJ19 DNAs were sequenced according to the dideoxy method and technology devised by Sanger et al (Sanger et al (1977), Proc. Natl. Acad. Sci. USA, 74 , 5463 and M13 cloning and sequencing handbook, AMERSHAM (1983). the 17-mer oligonucleotide primer $\alpha$-$^{35}$SdATP (400Ci/mmol, AMERSHAM), and 0.5X-5X buffer gradient gels (Biggen M.D. et al (1983, Proc. Natl. Acad. Sci. USA, 50, 3963) were used. Gels were read and put into the computer under the programs of Staden (Staden R. (1982), Nucl. Acids Res. 10, 4731). All the appropriate references and methods can be found in the AMERSHAM M13 cloning and sequencing handbook.

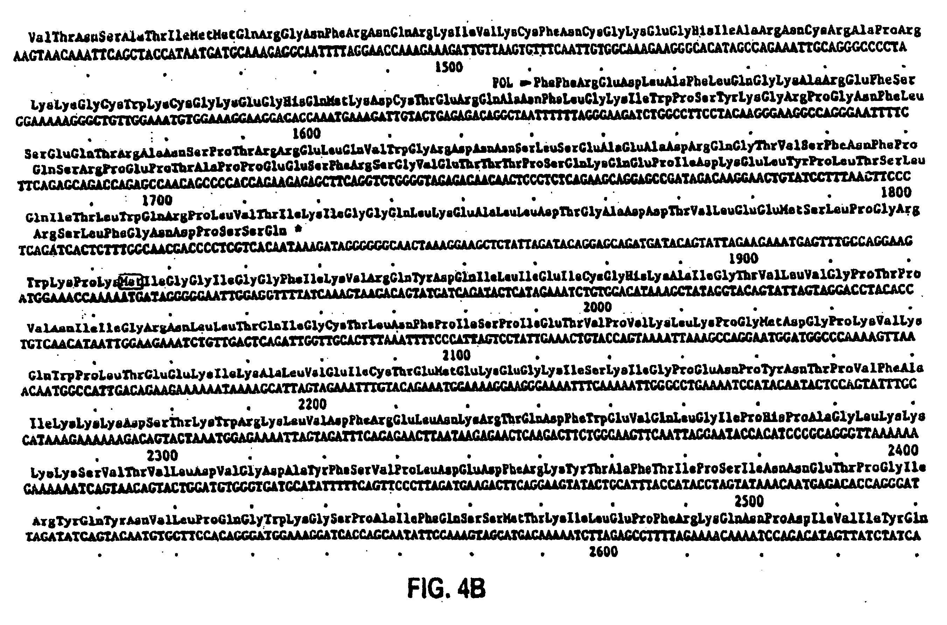

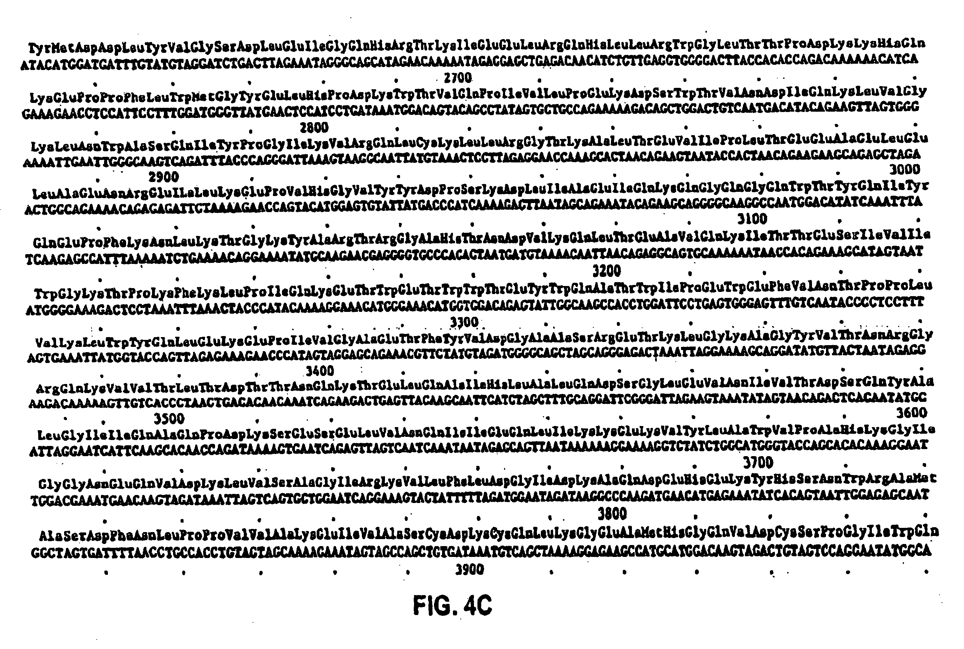

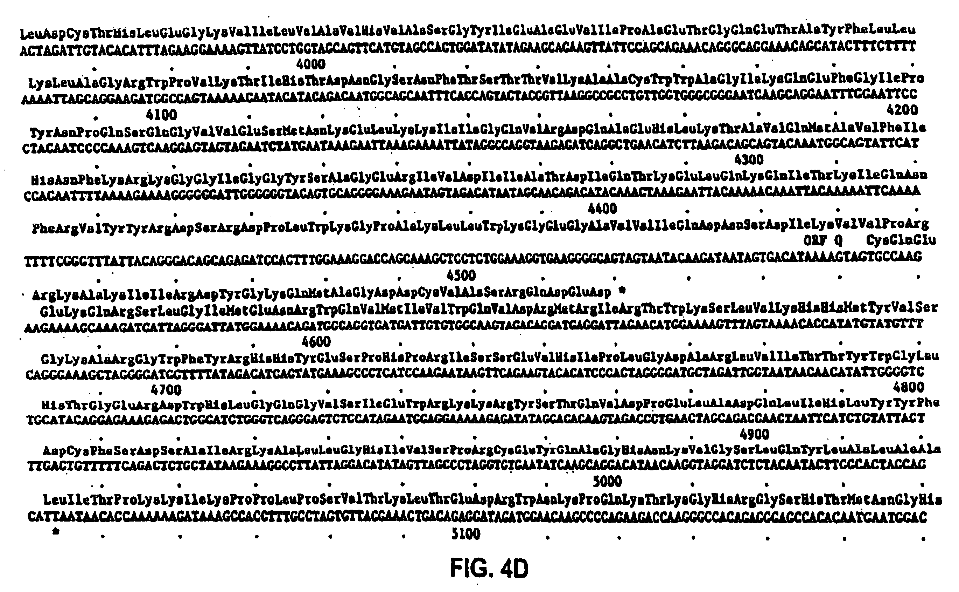

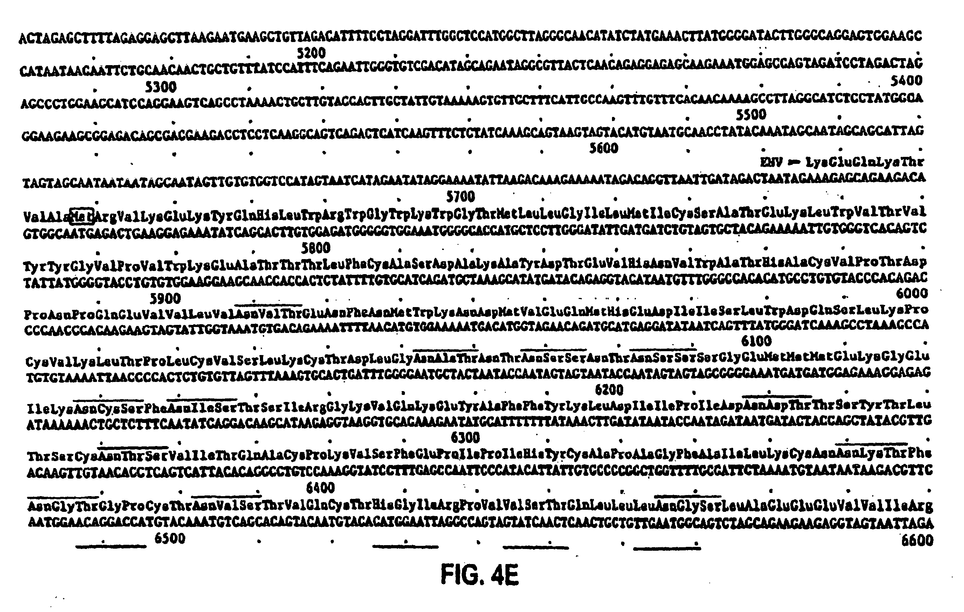

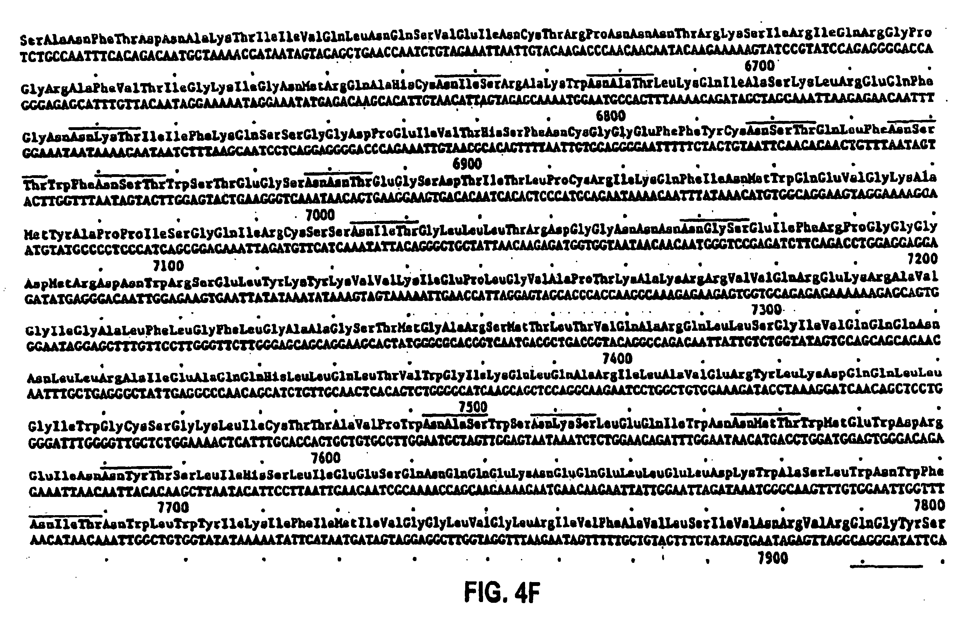

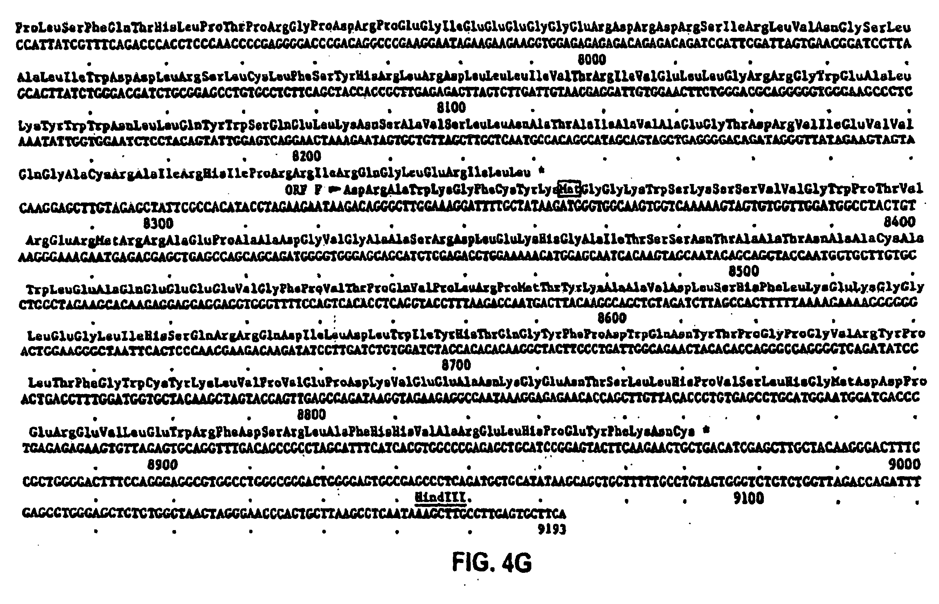

The complete DNA sequence of λJ19 (also designated as LAV-Ia) is shown in figs. 4 to 4e.

The sequence was reconstructed from the sequence of phage λJ19 insert. The numbering starts at the cap site which was located experimentally (see hereafter). Important genetic elements, major open reading frames and their predicted products are indicated together with the HindIII cloning sites. The potential glycosylation sites in the env gene are overlined. The $NH_2$-terminal sequence of p25$^{gag}$ determined by protein microsequencing is boxed.

Each nucleotide was sequenced on average 5.3 times : 85 % of the sequence was determined on both strands and the remainder sequenced at least twice from independent clones. The base composition is T, 22.2 % ; C, 17.8 % ; A, 35.8 % ; G, 244.2 % ; G + C, 42 %. The dinucleotide GC is greatly under represented (0,9 %) as common amongst eukaryotic sequences (Bird 1980).

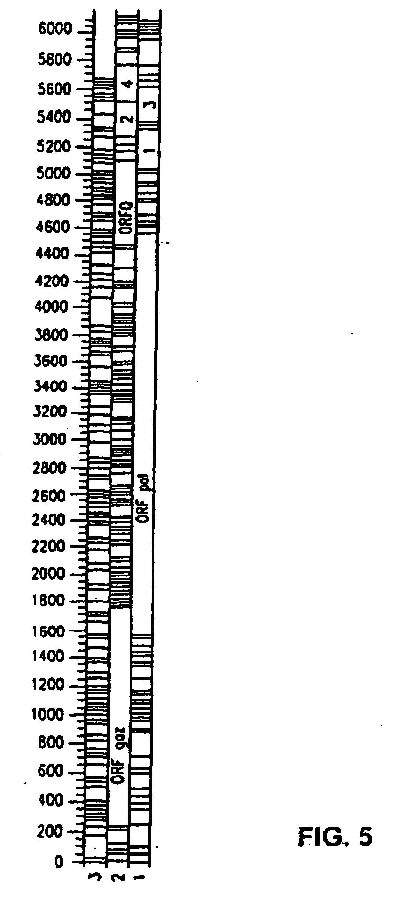

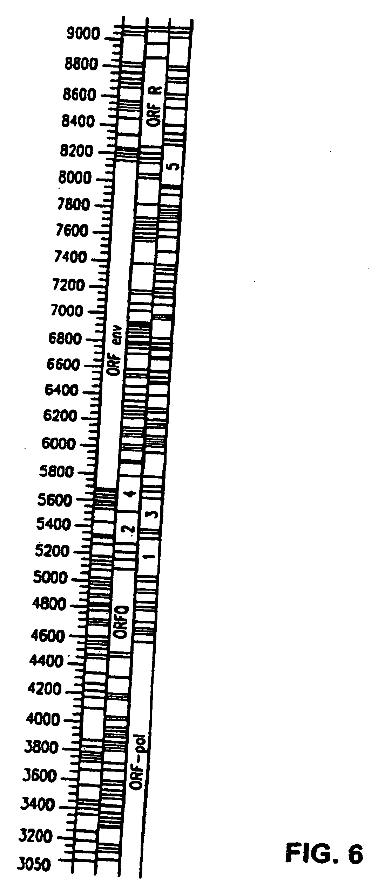

Figs. 5 and 6 provide a diagrammatized representation of the lengths of the successive open reading frames corresponding to the successive reading phases (also referred to by numbers "1", "2" and "3" appearing in the left handside part of fig. 5). The relative positions of these open reading frames (ORF) with respect to the nucleotidic structure of the LAV genome is referred to by the scale of numbers representative of the respective positions of the corresponding nucleotides in the DNA sequence. The vertical bars correspond to the positions of the corresponding stop codons.

The following genes and DNA fragments can be distinguished on the different reading frames shown. Reference is then also made to the proteins or glycoproteins encoded by said genes and fragments, , particularly to ORFs sequences such as ORF-0 and ORF-F.

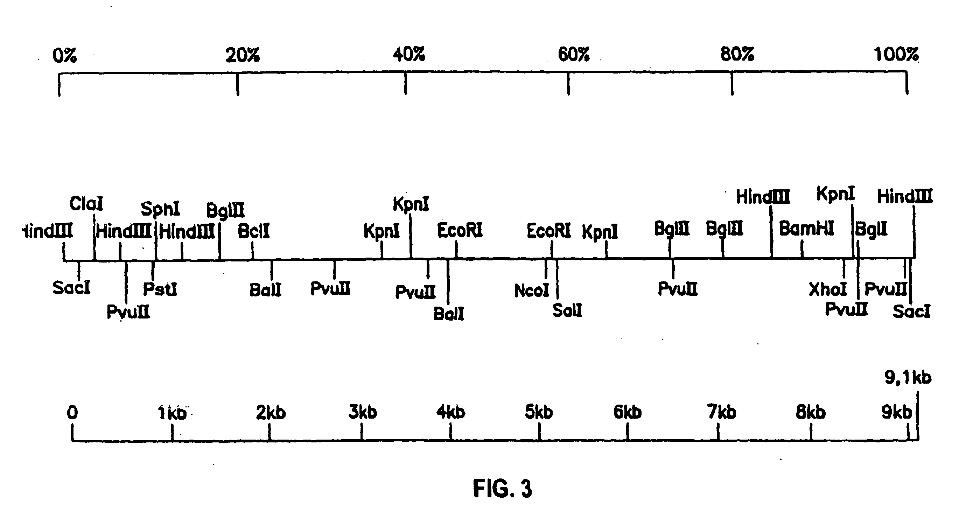

The application further discloses DNA sequences which provide open reading frames defined as ORF-Q, ORF-F and as "1", "2", "3", "4", "5", the relative positions of which appear more particularly in figs. 2 and 3. These ORFs have the following locations :

| ORF-Q | phase 1 | | start 4554 | | stop 5162 |
| --- | --- | --- | --- | --- | --- |
| ORF-F | " | 2 | " | 8324 | " | 8972 |
| ORF-1 | " | 1 | " | 5105 | " | 5392 |
| ORF-2 | " | 2 | " | 5349 | " | 5591 |
| ORF-3 | " | 1 | " | 5459 | " | 5692 |
| ORF-4 | " | 2 | " | 5595 | " | 5849 |
| ORF-5 | " | 1 | " | 8042 | " | 8355 |

## ORFs O and F

The viral (+) strand of the LAV genome was found to contain the statutory retroviral genes encoding the core structural proteins (gag), reverse transcriptase (pol) and envelope protein (env). and two extra open reading frames (orf) which we call O and F (Table 1). The genetic organization of LAV. 5'LTR-gag-pol-O-env-F-3'LTR, is unique. Whereas in all replication competent retroviruses pol and env genes overlap, in LAV they are separated by orf O (192 amino acids) followed by four small (<100 triplets) orf. The orf F (206 amino acids) slightly overlaps the 3' end of env and is remarkable in that it is half encoded by the U3 region of the LTR.

Such a structure places LAV clearly apart from previously sequenced retroviruses (Fig. 2). The (-) strand is apparently non coding. The additional HindIII site of the LAV clone λJ81 (with respect to λJ19) maps to the apparently non-coding region between Q and env (positions 5166-5745). Starting at position 5501 is a sequence (AAGCCT) which differs by a single base (underlined) from the HindIII recognition sequence. It is to be anticipated that many of the restriction site polymorphism between different isolates will map to this region. Clone λJ81 has also been referred to in British application Nr. 84 23659 filed on September 15, 1984.

O and F :

The nucleotide positions of their respective extremities are given in Table 1 hereafter.

The location of orf O is without precedent in the structure of retroviruses. Orf F is unique in that it is half encoded by the U3 element of the LTR. Both orfs have "strong" initiator codons (Kozak 1984) near their 5' ends and can encode proteins of 192 aminoacids (MW calc = 22487) and 206 aminoacids (MWcalc = 23316) respectively. Both putative proteins are hydrophilic (pO 49 % polar, 15.1 % Arg + Lys : pF 46 % polar, 11 % Arg + Lys) and are therefore unlikely to be associated directly with membrane. The function for the putative proteins pO and pF cannot be predicted as no homology was found by screening protein sequence data banks. Between orf F and the pX protein of HTLV-1 there is no detectable homology. Furthermore their hydrophobicity/hydrophilicity profiles are completely different. It is known that retroviruses can transduce cellular genes notably proto-oncogenes (Weinberg 1982)). We suggest that orfs O and F represent exogenous qenetic material and not some vestige of cellular DNA because (I) LAV DNA does not hybridize to the human genome under stringent conditions (Alizon et al., 1984), (II) their codon usage is comparable to that of the gag, pol and env genes (data not shown).

The organization of a reconstructed LTR and viral flanking elements are shown schematically in Fig. 6. The LTR is 638 bp long and displays usual features (Chen and Barker 1984) : (I) It is bounded by an inverted repeat (5'ACTG) including the conserved TG dinucleotide (Temin 1981). (II) Adjacent to 5' LTR is the tRNA primer binding site (PBS). complementary to $tRNA^{lys}_3$ (Raba et al., 1979).

(III) adjacent to 3'LTR is a perfect 15 bp polypurine tract. The other three polypurine tracts observed between nucleotides 8200-8800 are not followed by a sequence which is complementary to that just preceeding the PBS. The limits of U5, R and U3 elements were determined as follows. U5 is located between PBS and the polyadenylation site established from the sequence of the 3' end of oligo(dT)-primed LAVcDNA (Alizon et al., 1984). Thus U5 is 84 bos long. The length of R+U5 was determined bv synthesizing tRNA-primed LAV cDNA. After alkaline hydrolysis of the primer, R+U5 was found to be 181 ± 1 bp. Thus R is 97 bps long and the capping site at its 5' end can be located. Finally U3 is 456 bp long. The LAV LTR also contains characteristic regulatory elements : a polyadenylation signal sequence AATAAA 19 bp from the R-U5 junction and the sequence ATATAAG which is very likely the TATA box, 22 bps 5' of the cap site. There are no longer direct repeats within the LTR. Interestingly the LAV LTR shows some similarities to that of the mouse mammary tumour virus (MMTV) Donehower et al., 1981). They both use $tRNA^{lys}_3$ as a primer for (-) strand synthesis whereas all other exogenous mammalian retroviruses known to date use tRNA[pro] (Chen and Barker 1984). They possess very similar polypurine tracts (that of LAV is AAAAGAAAAGGGGGG while that of MMTV is AAAAAAGAAAAAGGGGG). It is probable that the viral (+) strand synthesis is discontinuous since the polypurine tract flanking the U3 element of the 3' LTR is found exactly duplicated in the 3' end of orf pol, at 4331-4336. In addition, MMTV and LAV are exceptionnal in that the U3 element can encode an orf. In the case of MMTV, U3 contains the whole orf while in LAV, U3 contains 110 codons of the 3' half of orf F.

The LAV long terminal repeat (LTR) is diaqrammatically represented in Fig. 6. As mentioned the LTR was reconstructed from the sequence of AJ19 by juxtaposing the sequences adiacent to the HindIII cloning sites.

Sequencing of oligo(dT) primed LAV DNA clone pLAV75 (Alizon et al., 1984) rules out the possibility of clustered HindIII sites in the R region of LAV. LTR are limited by an invertd repeat sequence (IR). Both of the viral elements flanking the LTR have been represented = tRNA primer binding site (PBS) for 5' LTR and polypurine track (PU) for 3' LTR. Also indicated are a putative TATA box, the cap site, polydenylation signal (AATAAA) and polyadenylation site (CAA). The location of the open reading frame F (648 nucleotides) is shown above the LTR schema.

The LTR (long terminal repeats) can also be defined as lying between position 8560 and position 160 (end extending over position 9097/1). As a matter of fact the end of the genome is at 9097 and, because of the LTR structure of the retrovirus, links up with the beginning of the sequence :

<div align="center">

**Hind III**

**CTCAATAAAGCTTGCCTTG**

↑↑

9097 1

</div>

Table 1 sums up the locations and sizes of viral open reading frames. The nucleotide coordinates refer to the first base of the first triplet (1st triplet), of the first methionine initiation codon (Met) and of the stop codon (stop). The number of aminoacids and calculated molecular weights are those calculated for unmodified precursor products starting at the first methionine through to the end with the exception of pol where the size and MW refer to that of the whole orf.

Tabe 1 : Location and sizes of viral open reading traines.

| orf | 1st triplet | Met | stop | No amino acids | MWcalc |
|---|---|---|---|---|---|
| gag | 312 | 336 | 1836 | 500 | 55841 |
| pol | 1631 | 1934 | 4640 | (1003) | (113629) |
| orf Q | 4554 | 4587 | 5163 | 192 | 22487 |
| env | 5746 | 5767 | 8350 | 861 | 97376 |
| orf F | 8324 | 8354 | 8972 | 206 | 23316 |

The invention concerns more particularly all the DNA fragments which have been more specifically referred to hereabove and which correspond to open reading frames. It will be understood that the man skilled in the art will be able to obtain them all, for instance by cleaving an entire DNA corresponding to the complete qenome of a LAV species, such as by cleavage by a partial or complete digestion thereof with a suitable restriction enzyme and by the subsequent recovery of the relevant fraqments. The different DNAs disclosed above can be resorted to also as a source of suitable fragments. The techniques disclosed hereafter for the isolation of the fragments which were then included in the plasmids referred to hereabove and which were then used for the DNA sequencing can be used.

Of course other methods can be used. Some of them have been examplified in British Application Nr. 8423659 filed on September 19, 1984. Reference is for instance made to the following methods.

a) DNA can be transfected into mammalian cells with appropriate selection markers by a variety of techniques, calcium phosphate precipitation, polyethylene glycol, protoplast-fusion, etc..
b) DNA fragments corresponding to genes can be cloned into expression vectors for E. coli . yeast- or mammalian cells and the resultant proteins purified.
c) The provival DNA can be "shot-gunned" (fragmented) into procaryotic expression vectors to generate fusion polypeptides. Recombinant producing antigenically competent fusion proteins can be identified by simply screening the recombinants with antibodies against LAV antigens.

The invention further refers more specifically to DNA recombinants, particularly modified vectors, including any of the preceding DNA sequences and adapted to transform corresponding microorganisms or cells, particularly eucaryotic cells such as yeasts, for instance Saccharomyces cerevisiae, or higher eucaryotic cells, particularly cells of mammals, and to permit expression of said DNA sequences in the corresponding microorganisms or cells.

More particularly the invention relates to such modified DNA recombinant vectors modified by the abovesaid DNA sequences and which are capable of transforming higher eucaryotic cells particularly mammalian cells. Preferably any of the abovesaid sequences are placed under the direct control of a promoter contained in said vectors and which is recognized by the polymerases of said cells, such that the first nucleotide codons expressed correspond to the first triplets of the above-defined DNA-sequences. Accordingly this invention also relates to the corresponding DNA fragments which can be obtained from LAV genomes or corresponding cDNAs by anv appropriate method. For instance such a method comprises cleaving said LAV genomes or cDNAs by restriction enzymes preferably at the level of restriction sites surrounding said fragments and close to the opposite extremities respectively thereof, recovering and identifying the fragments sought according to sizes, if need be checking their restriction maps or nucleotide sequences (or by reaction with monoclonal antibodies specifically directed against epitopes carried by the polypeptides encoded by said DNA fragments), and further if need be, trimming the extremities of the fragment, for instance by an exonucleolytic enzyme such as Bal31, for the purpose of controlling the desired nucleotide-sequences of the extremities of said DNA fragments or, conversely, repairing said extremities with Klenow enzyme and possibly ligating the latter to synthetic polynucleotide fragments designed to permit the reconstitution of the nucleotide extremities of said fragments. Those fragments may then be inserted in any of said vectors for causing the expression of the corresponding polypeptide by the cell transformed therewith. The corresponding polypeptide can then be recovered from the transformed cells, if need be after lysis thereof, and purified, by methods such as electrophoresis. Needless to say that all conventionnal methods for performing these operations can be resorted to.

The invention also relates more specifically to cloned probes which can be made starting from any DNA fragment according to this invention, thus to recombinant DNAs containing such fragments, particularly any plasmids amplifiable in procaryotic or eucaryotic cells and carrying said fragments.

Using the cloned DNA fragments as a molecular hybridization probe - either by labelling with radionucleotides or with fluorescent reagents - LAV virion RNA may be detected directly in the blood, body fluids and blood products (e.g.

of the antihemophylic factors such as Factor VIII concentrates) and vaccines, i.e. hepatitis B vaccine. It has already been shown that whole virus can be detected in culture supernatants of LAV producing cells. A suitable method for achieving that detection comprises immobilizing virus onto a support, e.g. nitrocellulose filters, etc., disrupting the virion and hybridizing with labelled (radiolabelled or "cold" fluorescent- or enzyme-labelled) probes. Such an approach has already been developed for Hepatitis B virus in peripheral blood (according to SCOTTO J. et al. Hepatology (1983), 3, 379-384).

Probes according to the invention can also be used for rapid screening of genomic DNA derived from the tissue of patients with LAV related symptoms, to see if the proviral DNA or RNA is present in host tissue and other tissues.

A method which can be used for such screening comprises the following steps : extraction of DNA from tissue, restriction enzyme cleavage of said DNA, electrophoresis of the fragments and Southern blotting of genomic DNA from tissues, subsequent hybridization with labelled cloned LAV proviral DNA. Hybridization in situ can also be used.

Lymphatic fluids and tissues and other non-lymphatic tissues of humans, primates and other mammalian species can also be screened to see if other evolutionnary related retrovirus exist. The methods referred to hereabove can be used, although hybridization and washings would be done under non stringent conditions.

The DNAs or DNA fragments according to the invention can be used also for achieving the expression of LAV viral antigens for diagnostic purposes.

The application discloses generally the polypeptides themselves, expressed by the different DNAs of the invention , particularly by the ORFs or fragments thereof, in appropriate hosts, particularly procaryotic or eucaryotic hosts, after transformation thereof with a suitable vector previously modified by the corresponding DNAs.

More generally, the application also discloses any of the polypeptide fragments (or molecules, particularly glycoproteins having the same polypeptidic backbone as the polypeptides mentioned hereabove) bearing an epitope characteristic of a LAV protein or glycoprotein, which polypeptide or molecule then has N-terminal and C-terminal extremities respectively either free or, independently from each other, covalently bound to aminoacids other than those which are normally associated with them in the larger polypeptides or glycoproteins of the LAV virus, which last mentioned aminoacids are then free or belong to another polypeptidic sequence. Particularly the application discloses hybrid polypeptides containing any of the epitope-bearing-polypeptides which have been defined more specifically hereabove, recombined with other polypeptides fragments normally foreign to the LAV proteins, having sizes sufficient to provide for an increased immunogenicity of the epitope-bearing-polypeptide yet, said foreign polypeptide fragments either being immunogenically inert or not interfering with the immunogenic properties of the epitope-bearing-polypeptide.

Such hybrid polypeptides which may contain up to 150, even 250 aminoacids usually consist of the expression products of a vector which contained ab initio a nucleic acid sequence expressible under the control of a suitable promoter or replicon in a suitable host, which nucleic acid sequence had however beforehand been modified by insertion therein of a DNA sequence encoding said epitope-bearing-polypeptide.

Said epitope-bearing-polypeptides, particularly those whose N-terminal and C-terminal aminoacids are free, are also accessible by chemical synthesis, according to technics well known in the chemistry of proteins.

The glycoproteins, proteins and polypeptides (generally designated hereafter as "antigens" of this invention, obtained by DNA recombinant technology are useful in processes for the detection of the presence of anti-LAV antibodies in biological media, particularly biological fluids such as sera from man or animal, particularly with a view of possibly diagnosing LAS or AIDS.

The peptides containing less than 250 aminoacid units, preferably less than 150; are deducible from the complete genomas of LAV.

The invention further relates to the hosts (procaryotic or eucaryotic cells) which are transformed bv the above mentioned recombinants and which are capable of expressing said DNA fragments.

Finally the invention also concerns vectors for the transformation of eucaryotic cells of human origin, particularly lymphocytes, the polymerases of which are capable of recognizing the LTRs of LAV. Particularly said vectors are characterized by the presence of a LAV LTR therein, said LTR being then active as a promoter enabling the efficient transcription and translation in a suitable host of a DNA insert coding for a determined protein placed under its controls.

Needless to say that the invention extends to all variants of genomes and corresponding DNA fragments (ORFs) having substantially equivalent properties, all of said genomes belonging to retroviruses which can be considered as equivalents of LAV.

It must be understood that the claims which follow are also intended to cover all equivalents of the products (glycoproteins, polypeptides, DNAs, etc..), whereby an equivalent is a product, i.e. a polypeptide which may distinguish from a determined one defined in any of said claims, say through one or several aminoacids, while still having substantially the same immunological or immunogenic properties. A similar rule of equivalency shall apply to the DNAs, it being understood that the rule of equivalency will then be tied to the rule of equivalency pertaining to the polypeptides which they encode.

It will also be understood that all the litterature referred to hereinbefore or hereinafter, and all patent applications or patents not specifically identified herein but which form counterparts of those specifically designated herein must be considered as incorporated herein by reference.

REFERENCES

Alizon, M., Sonigo, P., Barré-Sinoussi, F., Chermann, J.C., Tiollais, P., Montagnier, L. and Wain-Hobson, S. (1984). Molecular cloning of lymphadenopathy-associated virus. Nature, in press.

Arya, S.K., Gallo, R.C., Hahn, B.H., Shaw, G.M., Popovic, M., Salahuddin, S.Z. and Wong-Staal, F. (1984). Homology of genome of AIDS-associated virus with genomes of human T-cell leukemia lymphoma viruses. Science 225, 927-930.

Barré-Sinoussi, F., Chermann, J.C., Rey, F., Nugeybe, M.T., Chamaret, S., Gruest, J., Dauguet, C., Axler-Blin, C., Vézinet-Brun F., Rouzioux, C., Rozenbaum, W. and Montagnier, L. (1983). Isolation of a T-lymphotropic retrovirus from a patient at risk of Acquired Immune Deficiency Syndrome (AIDS). Science 220, 868-870.

Biggen, M.D., Gibson, T.J. and Hong, G.F. (1983). Buffer gradient gels and 35S label as an aid to rapid DNA sequence determination. Proc. Natl. Acad. Sci. USA 80, 3963-3965.

Bird, A.P. (1980). DNA methylation and the frequency of CpG in animal DNA. Nucl. Acids Res. 8, 1499-1504.

Brun-Vézinet, F., Rouzioux, C., Barré-Sinoussi, F., Klatzmann, D., Saimot, A.G., Rozembaum, W., Montagnier, L. and Chermann, J.C. (1984). Detection of IgG antibodies to lymphadenopathy associated virus (LAV) by ELISA, in patients with acquired immuno-deficiency syndrome of lymphadenopathy syndrome. Lancet I, 1253-1256.

Chen, H.R. and Barker, W.C. (1984). Nucleotide sequences of the retroviral long terminal repeats and their adjacent regions. Nucl. Acids Res. 12, 1767-1778.

Chen, I.S.Y., Mc Laughlin, J., Gasson, J.C., Clark, S.C. and Golde, D.W. (1983). Molecular characterization of genome of a novel human T-cell leukaemia virus. Nature 305, 502-505.

Chiu, I.M., Callahan, R., Tronick, S.R., Scholm, J. and Aaronson, S.A. (1984). Major pol gene progenitors in the evolution of oncornaviruses. Science 223, 364-370.

Cianciolo, G.J., Kipnis, R.J. and Snyderman, R. (1984). Similarity between p15E of murine and feline viruses and p2! of HTLV. Nature 311, 515.

Daly, H.M. and Scott, G.L. (1983). Fatal AIDS in a haemophiliae in the U.K. Lancet II, 1190.

Dittmar, K.J. and Moelling, K. (1973). Biochemical properties of p15-associated protease in an avion RNA tumor virus. J. Virol. 28, 106-113.

Donehower, L.A., Huang, A.L. and Hager, G.L. (1981). Regulatory and coding potential of the mouse mammary tumour virus long terminal redundancy. J. Virol. 37, 226-238.

Gottlieb, M.S., Schroff, R., Schanler, H.M., Weisman, J.D., Fan P.T., Wolf R.A., Saxon, A. (1981). Pneumocytis carinii pneumonia and mucosal candidiasis in previously healthy homosexual men : Evidence of a new acquired cellular immuno-deficiency. N.Engl. J. Med. 305, 1426-1431.

Hahn, B.H., Shaw, G.M., Arya, S.U., Popovic, M., Gallo, R.C. and Wong-Stall, F. (1984). Molecular cloning and characterization of the HTLV-III virus associated with AIDS. Nature 312, 166-169.

Harris, J.D., Scott, J.V., Taylor, B., Brahic, M., Stowring, L., Ventura, P., Haase, A.T. and Peluso, R. (1981). Visna virus DNA : discovery of a novel gapped structure. Virology 113, 573-583.

Kiyokawa, T., Yoshikura, H., Hattori, S., Secki, M. and Yoshida, M. (1984). Envelope proteins of human T-cell leukemia virus : expression in Escherischia coli and its- application to studies of env gene functions. Proc. Natl.

Acad. Sci. USA 81, 6202-6206.

Kiatzmann, D., Barré-Sinoussi, F., Nugeyre, M.T., Dauguet, C., Vilmer, E., Griscelli, C., Brun-Vézinet, F., Rouzioux, C., Gluckman, J.C., Chermann, J.C. and Montagnier, L. (1984). Selective tropism of lymphadenopathy associated virus (LAV) for helper-inducer T-lymphocytes. Sciences 225, 59-63.

Kozak, M. (1984). Compilation and analysis of sequences upstream from the transcriptional start site in eucaryotic mRNAs. Nucl. Acids Res. 12, 857-872.

Levy, J.A., Hoffman, A.D., Kramer, S.M., Lanois, J.A., Shimabukuro, J.M. and Oskiro, L.S. (1984). Isolation of lymphocytopathic retroviruses from San Francisco patients with AIDS. Science 225, 840-842.

Masur, H., Michelis, M.A., Greene, J.B., Onovato, I., Van de Stowe, R.A., Holzman, R.S., Wormser, G., Brettman, L., Lange, M., Murray, H.W., Cunningham-Rundles, S. (1981). An outbreak of community-acquired pneumocystis carinii pneumonia : Initial manifestation of cellular immune dysfunction. N. Engl. J. Med. 305, 1431-1438.

Misra, T.K., Grandgenett, D.P. and Parsons, J.T. (1982). Avian retrovirus pp32 DNA-binding protein. I. Recognition fo specific sequences on retrovirus DNA terminal repeats. J. Virol. 44, 330-343.

Montagnier, L., et al., (1984), A new human T-lymphotropic retrovirus : characterization and possible role in lymphadenopathy and acquired immune deficiency syndromes. In human T-cell leukemia/lymphoma viruses. R.C. Gallo, M. Essex and L. Gross, eds. (Cold Spring Laboratory, New-York), pp 363-370.

Oroszlan, S., Copeiand, T.D., Kalyanaraman, V.S., Sarngadharan, M.G., Schultz, A.M. and Gallo, R.C. (1984). Chemical analysis of human T-cell leukemia virus structural proteins. In HTLVS (R.C. Gallo, M.E. Essex and L. Gross, eds) Cold Spring Laboratory, New-York, pp 101-110.

Piot, P., Quinn, T.C., Taelmann, H., Feinsod, F.M. et al., (1984). Acquired immunodeficiency syndrome in a heterosexual population in Zaire. Lancet II, 65-69.

Popovic, M., Sarngadharan, M.G., Read, E. and Gallo, R.C. (1984). Detection, isolation, and continuous production of cytopathic retroviruses (HTLV-III) from patients with AIDS and pre-AIDS. Science 224, 497-500.

Querat, G., Barban, N., Sauze, N., Filippi, P., Vigne, R., Russo, P. and Vitu, C. (1984). Highly lytic and persistent lentiviruses naturally present in sheep with progressive pneumonia are genetically distinct. J. Virol. 52, 672-679.

Raba, M., Limburg, K., Burghagen, M., Katze, J.R., Simsek, M., Heckman, J.E., Rajbhandary, U.L., and Gross, H.J. (1979). Nucleotide sequence fo three isoaccepting lysine tRNAs from rabbit liver and SV40-transformed mouse fibroblasts. Eur. J. Biochem. 97, 305-318.

Rice, N.R., Stephens, R.M., Couez, D., Deschamps, J., Kettmann, R., Burny, A., and Gilden, R.V. (1984). The nucleotide sequence of the env gene and post-env region of bovine leukemia virus. Virology 138, 32-93.

Sagata, N., Yasunaga, T., Ogawa, Y., Tsuzuku-Kawamura, J. and Ikawa, Y. (1984). Bovine leukemia virus : Unique structural features of its long terminal repeats and its evolutionary relationship to human T-cell leukemia virus. Proc. Natl. Acad. Sci. USA 81, 4741-4745.

Sanger, F., Nicklen, S. and Coulsen, A.R. (1977). DNA sequencing with chain terminating inhibitors. Proc. Natl. Acad. Sci. USA 74, 5463-5467.

Schwartz, D.E., Tizard, R. and Gilbert, W. (1983). Nucleotide sequence of Rous sarcoma virus. Cell 32, 853-869.

Schüpbach, J., Popovic, M., Gilden, R.V., Gonda, M.A., Sarngadharan, M.G. and Gallo, R.C. (1984). Serological analysis of a subgroup of human T-lymphotropic retroviruses (HTLV-III) associated with AIDS. Science 224, 503-505.

Seiki, M., Hattori, S., Hirayama, Y. and Yoshida, M. (1983). Human adult T-cell leukemia virus : complete nucleotide sequence of the provirus genome integrated in leukemia cell DNA. Proc. Natl. Acad. Sci. USA 80, 3618-3622.

Shaw, G.M., Hahn, B.H., Arya, S.K., Groopman, J.E., Gallo, R.C. and Wong-Staal, F. (1984). Molecular characterization of human T-cell leukemia (lymphotropic) virus type III in the Acquired Immune Deficiency Syndrome. Science 226, 1165-1171.

Shimotohno, k., and Temin, H.M. (1982). Spontaneous variation and synthesis in the U3 region of the long terminal repeat of avion retroviruses. J. Virol. 41, 1636171.

Shimotohno, K., Golde, D.M., Miwa, M., Sugimura, T. and Chen, I.S.Y. (1984). Nucleotide sequence analysis fo the long terminal repeat of human T-cell leukemia virus type II. Proc. Natl. Acad. Sci. USA 31, 1079-1083.

Shinnick, T.M., Lerner, R.A. and Sutcliffe, J.G. (1981). Nucleotide sequence of Moloney murine leukemia viruse. Nature 293, 543-548.

Srinivasan, A., Reddy, E.P., Dunn, C.Y. and Aaronson, S.A. (1984). Molecular dissection of transcriptional control elements with the long terminal repeat of retrovirus. Science 223, 286-289.

Staden, R. (1982). Automation of the computer handling of gel reading data produced by the shotgun method of DNA sequencing. Nucl. Acids. Res. 10, 4731-4751.

Temin, H. (1981). Structure, variation and synthesis of retrovirus long terminal repeat. Cell 27, 1-3.

Weinberg, R.A. (1982). Fewer and fewer oncogenes. Cell 30, 3-9.

## Claims

1. DNA fragment from the DNA encoding the gag Open Reading Frame contained between nucleotide positions 312 and 1836 of the nucleic acid of figure 4, which comprises a fragment obtainable by cleavage with a suitable restriction enzyme.

2. DNA fragment according to claim 1, which is hybridizable with a restriction fragment obtainable from the nucleic acid of figure 4 contained between nucleotide positions 312 and 1836.

3. DNA fragment from the DNA encoding the gag Open Reading Frame contained between nucleotide positions 312 and 1836 of the nucleic acid of figure 4, which is suitable for use in a process for detection of a LAV virus.

4. DNA fragment from the DNA of the gag gene of LAV which comprises a restriction fragment obtainable from LAV DNA insert contained in pJ19.17 (I-367).

5. DNA fragment according to claim 4 which comprises a restriction fragment comprising at least one of the following restriction sites illustrated on the sequence of figure 3: HindIII, PvuII, PstI, SphI, HindIII.

6. DNA fragment according to claim 1, which is a cDNA fragment.

7. DNA fragment according to claim 1 or 2, which has from 300 to 600 bp.

8. DNA fragment according to claims 1 or 2, which is obtainable by cleavage with a suitable restriction enzyme and if appropriate by further fragmentation.

9. DNA fragment according to anyone of claims 1 to 6 which is the nucleic acid encoding the gag ORF represented on figure 4 between nucleotide positions 312 and 1836.

10. DNA fragment according to anyone of claims 1 to 9 which is a fragment of nucleic acid encoding the gag ORF represented on figure 4 between nucleotide positions 312 and 1836 and having at least one of the following restriction sites as represented on figure 3 :

    HindIII, PvuII, PstI, SphI, HindIII, BglII.

11. DNA fragment which is a variant of a DNA fragment according to anyone of claims 1 to 10, which distinguishes from said DNA fragment through one or several nucleotides, provided it encodes the same polypeptide.

12. DNA fragment according to anyone of claims 1 to 11 which is contained in a vector.

13. DNA fragment according to anyone of claims 1 to 11 which is a probe.

14. DNA fragment according to claim 1, encoding a polypeptide having sequence Pro Ile Val Gln Asn Ile Gln Gly Gln Met Val His Gln Ala Ile.

15. Probe according to claim 13 for the screening of genomic DNA derived from the tissue of patients with LAV related symptoms.

16. Use of a DNA fragment according to anyone of claims 1 to 11 for the screening of genomic DNA derived from the tissue of patients with LAV related symptoms.

17. Use of a DNA fragment according to anyone of claims 1 to 11 for the in vitro detection of viral LAV RNA in a body fluid obtained from a patient.

18. Use of a DNA fragment according to anyone of claims 1 to 11 for the detection of viral LAV RNA in blood products.

19. Process for the expression of a DNA fragment according to anyone of claims 1 to 11 comprising transforming a prokaryotic or a eukaryotic host cell with said DNA fragment, in conditions enabling its expression and recovering the expressed polypeptide.

**Patentansprüche**

1. DNA-Fragment der DNA, die den Offenen Leserahmen gag kodiert, der zwischen den Nukleotidpositionen 312 und 1836 der Nukleinsäure von Figur 4 enthalten ist, die ein mittels Schneidens mit einem geeigneten Restriktionsenzym erhältliches Fragment umfasst.

2. DNA-Fragment nach Anspruch 1, das mit einem Restriktionsfragment hybridisierbar ist, das von der Nukleinsäure der Figur 4 erhältlich ist, die zwischen den Nukleotidpositionen 312 und 1836 enthalten ist.

3. DNA-Fragment der DNA, die den zwischen den Nukleotidpositionen 312 und 1836 der Nukleinsäure von Figur 4 enthaltenen Offenen Leserahmen gag kodiert, das für die Verwendung in einem Verfahren zum Nachweis eines LAV-Virus geeignet ist.

4. DNA-Fragment der DNA des gag-Gens von LAV, das ein Restriktionsfragment umfasst, das von dem in pJ19.17 (I-367) enthaltenen LAV-DNA-Insert erhältlich ist.

5. DNA-Fragment nach Anspruch 4, das ein Restriktionsfragment umfasst, das wenigstens eine der folgenden, auf der Sequenz von Figur 3 dargestellten Restriktionsstellen umfasst: HindIII, PvuII, PstI, SphI, HindIII.

6. DNA-Fragment nach Anspruch 1, das ein cDNA-Fragment ist.

7. DNA-Fragment nach Anspruch 1 oder 2, das von 300 bis 600 bp hat.

8. DNA-Fragment nach Anspruch 1 oder 2, das mittels Schneidens mit einem geeigneten Restriktionsenzym und falls angebracht mittels weiterer Fragmentierung erhältlich ist.

9. DNA-Fragment nach einem beliebigen der Ansprüche 1 bis 6, das die Nukleinsäure ist, die den in Figur 4 zwischen den Nukleotidpositionen 312 und 1836 dargestellten gag-ORF kodiert.

10. DNA-Fragment nach einem beliebigen der Ansprüche 1 bis 9, das ein Nukleinsäurefragment ist, das den in Figur 4 zwischen den Nukleotidpositionen 312 und 1836 wiedergegebenen gag-ORF kodiert und wenigstens eine der folgenden wie in Figur 3 wiedergegebenen Restiktionsstellen aufweist:

HindIII, PvuII, PstI, SphI, HindIII, BglII.

**11.** DNA-Fragment, das eine Variante eines DNA-Fragments nach einem beliebigen der Ansprüche 1 bis10 ist, das sich von besagtem DNA-Fragment durch eines oder mehrere Nukleotide unterscheidet, vorausgesetzt, es kodiert dasselbe Polypeptid.

**12.** DNA-Fragment nach einem beliebigen der Ansprüche 1 bis 11, das in einem Vektor enthalten ist.

**13.** DNA-Fragment nach einem beliebigen der Ansprüche 1 bis 11, das eine Sonde ist.

**14.** DNA-Fragment nach Anspruch 1, das ein Polypeptid kodiert, das die Sequenz Pro Ile Val Gln Asn Ile Gln Gly Gln Met Val His Gln Ala Ile hat.

**15.** Sonde nach Anspruch 13 für das Screening genomischer DNA, die von dem Gewebe von Patienten mit LAV-verwandten Symptomen abgeleitet ist.

**16.** Verwendung eines DNA-Fragments nach einem beliebigen der Ansprüche 1 bis 11 für das Screening genomischer DNA, die von dem Gewebe von Patienten mit LAV-verwandten Symptomen abgeleitet ist.

**17.** Verwendung eines DNA-Fragments nach einem beliebigen der Ansprüche 1 bis 11 für den <u>in-vitro</u>-Nachweis viraler LAV-RNA in einer von einem Patienten erhaltenen Körperflüssigkeit.

**18.** Verwendung eines DNA-Fragments nach einem beliebigen der Ansprüche 1 bis 11 für den Nachweis viraler LAV-RNA in Blutprodukten.

**19.** Verfahren zur Expression eines DNA-Fragments nach einem beliebigen der Ansprüche 1 bis 11, umfassend Transformieren einer prokaryontischen oder eukaryontischen Wirtszelle mit besagtem DNA-Fragment unter Bedingungen, die seine Expression ermöglichen, und Gewinnen des exprimierten Polypeptids.

## Revendications

**1.** Fragment d'ADN provenant de l'ADN codant pour le cadre de lecture ouvert gag contenu entre les positions 312 et 1836 des nucléotides de l'acide nucléique de la figure 4, qui comprend un fragment susceptible d'être obtenu par clivage avec une enzyme de restriction appropriée.

**2.** Fragment d'ADN selon la revendication 1, qui est hybridable avec un fragment de restriction susceptible d'être obtenu à partir de l'acide nucléique de la figure 4 contenu entre les nucléotides 312 et 1836.

**3.** Fragment d'ADN provenant de l'ADN codant pour le cadre de lecture ouvert gag contenu entre les positions 312 et 1836 des nucléotides de l'acide nucléique de la figure 4, qui est approprié pour l'utilisation dans un procédé de détection d'un virus LAV.

**4.** Fragment d'ADN provenant de l'ADN du gène gag de LAV, qui comprend un fragment de restriction susceptible d'être obtenu à partir d'un insert d'ADN de LAV contenu dans pJ1917 (I-367).

**5.** Fragment d'ADN selon la revendication 4, qui comprend un fragment de restriction comprenant au moins un des sites de restriction suivants illustrés sur la séquence de la figure 3 : HindIII, PvuII, PstI, SphI, HindIII.

**6.** Fragment d'ADN selon la revendication 1, qui est un fragment d'ADNc.

**7.** Fragment d'ADN selon la revendication 1 ou 2, qui a de 300 à 600 pb.

**8.** Fragment d'ADN selon les revendications 1 ou 2, que l'on peut obtenir par clivage avec une enzyme de restriction appropriée et, si c'est approprié, par une fragmentation supplémentaire.

**9.** Fragment d'ADN selon l'une quelconque des revendications 1 à 6 qui est l'acide nucléique codant pour le cadre de lecture ouvert gag représenté à la figure 4 entre le nucléotide en position 312 et le nucléotide en position 1836.

**10.** Fragment d'ADN selon l'une quelconque des revendications 1 à 9, qui est un fragment d'acide nucléique codant pour le cadre de lecture ouvert (ORF) gag représenté à la figure 4, entre les positions 312 et 1836 des nucléotides, et ayant au moins un des sites de restriction suivants, tel que représenté à la figure 3 :

HindIII, PvuII, PstI, SphI, HindIII, BgIII.

**11.** Fragment d'ADN qui est un variant d'un fragment d'ADN selon l'une quelconque des revendications 1 à 10, qui se distingue dudit fragment d'ADN par un ou plusieurs nucléotides, à condition qu'il code pour le même polypeptide.

**12.** Fragment d'ADN selon l'une quelconque des revendications 1 à 11, qui est contenu dans un vecteur.

**13.** Fragment d'ADN selon l'une quelconque des revendications 1 à 11, qui est une sonde.

**14.** Fragment d'ADN selon la revendication 1, codant un polypeptide ayant la séquence Pro Ile Val Gln Asn Ile Gln Gly Gln Met Val His Gln Ala Ile.

**15.** Sonde selon la revendication 13 pour le dépistage d'ADN génomique provenant du tissu de patients ayant des symptômes liés à LAV.

**16.** Utilisation d'un fragment d'ADN selon l'une quelconque des revendications 1 à 11, pour le criblage de l'ADN génomique provenant du tissu de patients ayant des symptômes liés à LAV.

**17.** Utilisation d'un fragment d'ADN selon l'une quelconque des revendications 1 à 11, pour la détection *in vitro* de l'ARN viral dans un fluide corporel obtenu d'un patient.

**18.** Utilisation d'un fragment d'ADN selon l'une quelconque des revendications 1 à 11, pour la détection de l'ARN viral de LAV dans les produits sanguins.

**19.** Procédé pour l'expression du fragment d'ADN selon l'une quelconque des revendications 1 à 11 comprenant la transformation d'un hôte cellulaire procaryote ou eucaryote avec ledit fragment d'ADN, dans des conditions autorisant son expression et la récupération du polypeptide exprimé.

FIG. I

FIG. 2

λJ19

H + H − H | + H + H

pLAV13

H B XK SH
H
Bg Bg Bg

λJ81

H + H − H − H + H + H

λJ19

HS H P H K K R R Sa K H XK SH
B B Bg Bg Bg B Bg Bg

0 1 2 3 4 5 6 7 8 9 9.2kb

R U5 U3 R

H=HindIII    K=Kpn I
S=Sac I      R=EcoRI
B= Bam HI    Sa=Sal I
P=Pst I      X=Xho I
Bg=Bgl II

EP 1 231 271 B2

EP 1 231 271 B2

0%     20%     40%     60%     80%     100%

ClaI        SphI    BglII                    KpnI
HindIII   HindIII HindIII   BclI                      EcoRI        EcoRI  KpnI                         HindIII      KpnI    HindIII
                                              KpnI                                  BglII    BglII    BamHI         BglI

SacI              PstI         BalI    PvuII        PvuII    NcoI         PvuII          XhoI   PvuII
     PvuII                                   BalI         SalI             PvuII          SacI

9,1kb

0       1kb     2kb     3kb     4kb     5kb     6kb     7kb     8kb     9kb

FIG. 3

CGTCTCTC**GTTAGACCAGATTTGAGCCTGGCAGCCTCTCTGCCTAACTAGCGAACCCACTCCTTAAGCCTCAATAAACGTTGCCTTGAGTGCTTCAACTACTGTGTGCCCGTCTCTTCT

GTCACTGTGGTAACTAGACATCCCTCAGACCCTTTTAGTCAGTGTCGAAAATCTCTACCAGTGCCGCCCGAACAGCGACTTGAAAGCCGAAAGCGAAACCAGAGGAGCTCTCTCGACGCAG
                                                                                                                    200
                                                 GAG ► LeuAlaGluAlaArgArgArg[Met]GlyAlaArgAlaSerValLeuSer
GACTCGGCTTGCTGAAGCGCGCACCGGCAACAGCCGAGCCGAGCGCCACTGCTGACTACGCCAAAAATTTTGACTAGCCGGAGCGCTAGAAGCGAGAGAGATGCGTCCGACAGCCGTCAGTATTAA
                                                 300
  GlyGlyGluLeuAspArgTrpGluLysIleArgLeuArgProGlyGlyLysLysLysTyrLysLeuLysHisIleIleValTrpAlaSerArgGluLeuGluArgPheAlaValAsnProGly
  CCGGCGCCAGAATTAGATCCGATGGCAAAAAATTCGGTTAAGGCCCACGGGCAAACAAAAAATATAAATTAAAACATATAGTATGCGCAAGCAGCGAGCTAGAACGATTCGCAGTTAATCCTC
                    400
  LeuLeuGluThrSerGluGlyCysArgGlnIleLeuGlyGlnLeuGlnProSerLeuGlnThrGlySerGluGluLeuArgSerLeuTyrAsnThrValAlaThrLeuTyrCysValHis
  GCCTGTTAGAAACATCAGAAGGCTGTAGACAAATACTGGGACAGCTACAAGCCATCCCTTCAGACAGGATCAGAAGAAGTTAGATCATTATATAATACAGTAGCAACCCTCTATTGTGTGC
                    500                                                                                                      600
  GlnArgIleGluIleLysAspThrLysGluAlaLeuAspLysIleGluGluGluGlnAsnLysSerLysLysLysAlaGlnGlnAlaAlaAlaAspThrGlyHisSerSerGlnValSer
  ATCAAAGGATAGAGATAAAAGACACCAAGGAAGCTTTAGACAAGATAGAGGAAGAGCAAAACAAAAGTAAGAAAAAAGCACAGCAAGCAGCAGCTGACACAGGACACAGCAGCCAGGTCA
                                                                                                700
  GlnAsnTyr[ProIleValGlnAsnIleGlnGlyGlnMetValHisGlnAlaIle]SerProArgThrLeuAsnAlaTrpValLysValValGluGluLysAlaPheSerProGluValIle
  GCCAAAATTACCGTATAGTGCAGAACATCCAGGGGCAAATGGTACATCAGGCCATATCACCTAGAACTTTAAATGCATCGGTAAAAGTAGTAGAAGAGAAGGCTTTCAGCCCAGAAGTCA
                                                                 800
  ProMetPheSerAlaLeuSerGluGlyAlaThrProGlnAspLeuAsnThrMetLeuAsnThrValGlyGlyHisGlnAlaAlaMetGlnMetLeuLysGluThrIleAsnGluGluAla
  TACCCATGTTTTCAGCATTATCAGAAGGAGCCACCCCACAAGATTTAAACACCATGCTAAACACAGTGGGGGGACATCAAGCAGCCATGCAAATGTTAAAAGAGACCATCAATGAGGAAG
                                                900
  AlaGluTrpAspArgValHisProValHisAlaGlyProIleAlaProGlyGlnMetArgGluProArgGlySerAspIleAlaGlyThrThrSerThrLeuGlnGluGlnIleGlyTrp
  CTGCAGAATGGGATAGAGTGCATCCAGTGCATGCAGGGCCTATTGCACCAGGCCAGATGAGAGAACAACCAAGCGGAAGTGACATAGCAGGAACTACTAGTACCCTTCAGGAACAAATACGAT
                            1000
  MetThrAsnAsnProProIleProValGlyGluIleTyrLysArgTrpIleIleLeuGlyLeuAsnLysIleValArgMetTyrSerProThrSerIleLeuAspIleArgGlnGlyPro
  GGATGACAAATAATCCACCTATCCCAGTAGGAGAAATTTATAAAAGATGGATAATCCTGGGATTAAATAAAATAGTAAGAATGTATAGCCCTACCAGCATTCTGGACATAAGACAAGGAC
                    1100                                                                                            1200
  LysGluProPheArgAspTyrValAspArgPheTyrLysThrLeuArgAlaGluGlnAlaSerGlnGluValLysAsnTrpMetThrGluThrLeuLeuValGlnAsnAlaAsnProAsp
  CAAAAGAACCCTTTAGAGACTATGTAGACCCGTTCTATAAAACTCTAAGAGCCGAGCAAGCTTCACAGGAGGTAAAAAATTGGATGACAGAAACCTTGTTGGTCCAAAATGCGAACCCAG
                                                                    1300
  CysLysThrIleLeuLysAlaLeuGlyProAlaAlaThrLeuGluGluMetMetThrAlaCysGlnGlyValGlyGlyProGlyHisLysAlaArgValLeuAlaGluAlaMetSerGln
  ATTGTAAGACTATTTTAAAAGCATTGGGACCAGCAGCTACACTAGAAGAAATGATGACAGCATGTCAGGGAGTGGGAGGACCCGGCCATAAGGCAAGAGTTTTGGCTGAAGCAATGAGCC
                                        1400

ValThrAsnSerAlaThrIleMetMetGlnArgGlyAsnPheArgAsnGlnArgLysIleValLysCysPheAsnCysGlyLysGluGlyHisIleAlaArgAsnCysArgAlaProArg
AAGTAACAAATTCAGCTACCATAATGATGCAAACAGCCAATTTTAGGAACCAAAGAAAGATTGTTAAGTGTTTCAATTGTGGCAAAGAAGGGCACATAGCCAGAAATTGCAGGGCCCCTA
1500

POL → PhePheArgGluAspLeuAlaPheLeuGlnGlyLysAlaArgGluPheSer
LysLysGlyCysTrpLysCysGlyLysGluGlyHisGlnMetLysAspCysThrGluArgGlnAlaAsnPheLeuGlyLysIleTrpProSerTyrLysGlyArgProGlyAsnPheLeu
CGAAAAAGGGCTGTTGGAAATGTGGAAAGGAAGGACACCAAATGAAAGATTGTACTGAGAGACAGGCTAATTTTTTAGGGAAGATCTGGCCTTCCTACAAGGGAAGGCCAGGGAATTTTC
1600

SerGluGlnThrArgAlaAsnSerProThrArgArgGluLeuGlnValTrpGlyArgAspAsnAsnSerLeuSerGluAlaGluAlaAspArgGlnGlyThrValSerPheAsnPhePro
GlnSerArgProGluProThrAlaProProGluGluSerPheArgSerGlyValGluThrThrThrProSerGlnLysGlnGluProIleAspLysGluLeuTyrProLeuThrSerLeu
TTCAGAGCAGACCAGAGCCAACAGCCCCACCAGAAGAGAGCCTTCAGGTCTGGGGTAGAGACAACAACTCCGTCTCAGAAGCAGGAGCCGATAGACAAGGAACTGTATCCTTTAACTTCCC
1700                                                                                                                1800

GlnIleThrLeuTrpGlnArgProLeuValThrIleLysIleGlyGlyGlnLeuLysGluAlaLeuLeuAspThrGlyAlaAspAspThrValLeuGluGluMetSerLeuProGlyArg
ArgSerLeuPheGlyAsnAspProSerSerGln *
TCAGATCACTCTTTGGCAACGACCCCTCGTCACAATAAAGATAGGGGGGCAACTAAAGGAAGCTCTATTAGATACAGGAGCAGATGATACAGTATTAGAAGAAATGAGTTTGCCAGGAAG
1900

TrpLysProLysMetIleGlyGlyIleGlyGlyPheIleLysValArgGlnTyrAspGlnIleLeuIleGluIleCysGlyHisLysAlaIleGlyThrValLeuValGlyProThrPro
ATGGAAACCAAAAATGATAGGGGCAATTGGAGGTTTTATCAAAGTAAGACACAGTATCATCAGATACTCATAGAAATCTGTGGACATAAAGCTATAGGTACAGTATTAGTAGGACCTACACC
2000

ValAsnIleIleIleGlyArgAsnLeuLeuThrGlnIleGlyCysThrLeuAsnPheProIleSerProIleGluThrValProValLysLeuLysProGlyMetAspGlyProLysValLys
TGTCAACATAATTGGAAGAAATCTGTTGACTCAGATTGGTTGCACTTTAAATTTTCCCATTAGTCCTATTGAAACTGTACCAGTAAAATTAAAGCCAGGAATGGATGGCCCAAAAGTTAA
2100

GlnTrpProLeuThrGluGluLysIleLysAlaLeuValGluIleCysThrGluMetGluLysGluGlyLysIleSerLysIleGlyProGluAsnProTyrAsnThrProValPheAla
ACAATGGCCATTGACAGAAGAAAAAATAAAAGCATTAGTAGAAATTTGTACAGAAATGGAAAAGGAAGGGAAAATTTCAAAAATTCGGCCTGAAAATCCATACAATACTCCAGTATTTGC
2200

IleLysLysLysAspSerThrLysTrpArgLysLeuValAspPheArgGluLeuAsnLysArgThrGlnAspPheTrpGluValGlnLeuGlyIleProHisProAlaGlyLeuLysLys
CATAAAGAAAAAAGACAGTACTAAATGGAGAAAATTAGTAGATTTCAGAGAACTTAATAAGAGAACTCAAGACTTCTGGGAAGTTCAATTAGGAATACCACATCCCGCAGGGTTAAAAAA
2300                                                                                                                2400

LysLysSerValThrValLeuAspValGlyAspAlaTyrPheSerValProLeuAspGluAspPheArgLysTyrThrAlaPheThrIleProSerIleAsnAsnGluThrProGlyIle
GAAAAAATCAGTAACAGTACTGGATGTGGGTGATGCATATTTTTCAGTTCCCTTAGATGAAGACTTCAGGAAGTATACTGCATTTACCATACCTAGTATAAACAATGAGACACCAGGGAT
2500

ArgTyrGlnTyrAsnValLeuProGlnGlyTrpLysGlySerProAlaIlePheGlnSerSerMetThrLysIleLeuGluProPheArgLysGlnAsnProAspIleValIleTyrGln
TAGATATCAGTACAATGTGCTTCCACAGGGATGGAAAGGATCACCAGCAATATTCCAAAGTAGCATGACAAAAATCTTAGAGCCTTTTAGAAAACAAAATCCAGACATAGTTATCTATCA
2600

FIG. 4B

EP 1 231 271 B2

TyrMetAspAspLeuTyrValGlySerAspLeuGluIleGlyGlnHisArgThrLysIleGluGluLeuArgGlnHisLeuLeuArgTrpGlyLeuThrThrProAspLysLysHisGln
ATACATGGATGATTTGTATGTAGGATCTGACTTAGAAATAGGGCAGCATAGAACAAAAATAGACGAGCTGAGACAACATCTCTTGAGGTGGGGACTTACCACACCAGACAAAAAACATCA

2700

LysGluProProPheLeuTrpMetGlyTyrGluLeuHisProAspLysTrpThrValGlnProIleValLeuProGluLysAspSerTrpThrValAsnAspIleGlnLysLeuValGly
GAAAGAACCTCCATTCCTTTGGATGGCGTTATGAACTCCATCCTGATAAATCGACAGTACAGCCTATAGTGCTGCCAGAAAAAGACAGCTGGACTGTCAATGACATACAGAAGTTAGTCGGG

2800

LysLeuAsnTrpAlaSerGlnIleTyrProGlyIleLysValArgGlnLeuCysLysLeuLeuArgGlyThrLysAlaLeuThrGluValIleProLeuThrGluGluAlaGluLeuGlu
AAAATTGAATTGGGCAAGTCAGATTTACCCAGGGATTAAAGTAAGGCCAATTATGTAAACTCCTTAGAGGAACCAAAGCACTAACAGAAGTAATACCACTAACAGAAGAAGCAGAGCTAGA

2900                                                                                                    3000

LeuAlaGluAsnArgGluIleLeuLysGluProValHisGlyValTyrTyrAspProSerLysAspLeuIleAlaGluIleGlnLysGlnGlyGlnGlyGlnTrpThrTyrGlnIleTyr
ACTGGCAGAAAACAGAGAGATTCTAAAAGAACCAGTACATGGAGTGTATTATGACCCATCAAAAGACTTAATAGGCAGAAATACAGAAGCAGGGGGCCAATGGACATATCAAATTTA

3100

GlnGluProPheLysAsnLeuLysThrGlyLysTyrAlaArgThrArgGlyAlaHisThrAsnAspValLysGlnLeuThrGluAlaValGlnLysIleThrThrGluSerIleValIle
TCAAGAGCCATTTAAAAATCTGAAAACAGGAAAATATGCAAGAACGAGCGGGTGCCCACAGTAATCATGTAAAACAATTAACAGAGGCAGTGCAAAAAATAACCACAGAAAGCATAGTAAT

3200

TrpGlyLysThrProLysPheLysLeuProIleGlnLysGluThrTrpGluThrTrpTrpThrGluTyrTrpGlnAlaThrTrpIleProGluTrpGluPheValAsnThrProProLeu
ATGGGGAAAGACTCCTAAATTTAAACTACCCATACAAAAGGAAACATCGGAAACATGGTCGACAGAGTATTGGCAAGCCACCTGGATTCCTGAGTGGGAGTTTGTCAATACCCCTCCTTT

3300

ValLysLeuTrpTyrGlnLeuGluLysGluProIleValGlyAlaGluThrPheTyrValAspGlyAlaAlaSerArgGluThrLysLeuGlyLysAlaGlyTyrValThrAsnArgGly
AGTCAAATTATGGTACCAGTTAGAGAAAGAACCCATAGTAGGAGCAGAAACGTTCTATGTAGATGGGGCAGCTAGCAGGGAGACTAAAATTAGGAAAAGCAGGATATGTTACTAATAGAGG

3400

ArgGlnLysValValThrLeuThrAspThrThrAsnGlnLysThrGluLeuGlnAlaIleHisLeuAlaLeuGlnAspSerGlyLeuGluValAsnIleValThrAspSerGlnTyrAla
AAGACAAAAAGTTGTCACCCTAACTGACACAACAAATCAGAAGACTGAGTTACAAGCAATTCATCTAGCTTTGCAGGATTCGGGATTAGAAGTAAATATAGTAACAGACTCACAATATGC

3500                                                                                                    3600

LeuGlyIleIleGlnAlaGlnProAspLysSerGluSerGluLeuValAsnGlnIleIleGluGlnLeuIleLysLysGluLysValTyrLeuAlaTrpValProAlaHisLysGlyIle
ATTAGGAATCATTCAAGCACAACCAGATAAAACTGAATCAGAGTTAGTCAATCAAATAATAGAGCAGTTAATAAAAAAGGAAAAGGTCTATCTGGCATGGGTACCAGCACACAAAGGAAT

3700

GlyGlyAsnGluGlnValAspLysLeuValSerAlaGlyIleArgLysValLeuPheLeuAspGlyIleAspLysAlaGlnAspGluHisGluLysTyrHisSerAsnTrpArgAlaMet
TGGACGAAATGAACAAGTAGATAAATTAGTCAGTGCTGGAATCAGGAAAGTACTATTTTTAGATGGAATAGATAAGGCCCAAGATGAACATGAGAAATATCACAGTAATTCGACAGCAAT

3800

AlaSerAspPheAsnLeuProProValValAlaLysGluIleValAlaSerCysAspLysCysGlnLeuLysGlyGluAlaMetHisGlyGlnValAspCysSerProGlyIleTrpGln
GGCTAGTGATTTTAACCTGCCACCTGTAGTAGCAAAAGAAATAGTAGCCAGCTGTGATAAATGTCAGCTAAAAGGAGAAGCCATGCATGGACAAGTAGACTGTAGTCCAGGAATATGGCA

3900

FIG. 4C

LeuAspCysThrHisLeuGluGlyLysValIleLeuValAlaValHisValAlaSerGlyTyrIleGluAlaGluValIleProAlaGluThrGlyGlnGluThrAlaTyrPheLeuLeu
ACTAGATTGTACACATTTAGAAGGAAAAGTTATCCTGGTACCAGTTCATGTAGCCACTGGATATATAGAAGCAGAAGTTATTCCAGCAGAAACAGGGCAGGAAACAGCATACTTTCTTTT
                                    4000

LysLeuAlaGlyArgTrpProValLysThrIleHisThrAspAsnGlySerAsnPheThrSerThrThrValLysAlaAlaCysTrpTrpAlaGlyIleLysGlnGluPheGlyIlePro
AAAATTAGCAGGAAGATCGCCAGTAAAAACAATACATACAGACAATGGCAGCAATTTCACCAGTACTACCGGTTAAGGCCGCCTGTTGGTGGGCGGGAATCAAGCAGGAATTTGGAATTCC
    4100                                                                                                    4200

TyrAsnProGlnSerGlnGlyValValGluSerMetAsnLysGluLeuLysLysIleIleGlyGlnValArgAspGlnAlaGluHisLeuLysThrAlaValGlnMetAlaValPheIle
CTACAATCCCCAAAGTCAAGGAGTAGTAGAATCTATGAATAAAGAATTAAAGAAAATTATAGGCCAGGTAAGAGATCAGGCTGAACATCTTAAGACAGCAGTACAAATGGCAGTATTCAT
                                                                                    4300

HisAsnPheLysArgLysGlyGlyIleGlyGlyTyrSerAlaGlyGluArgIleValAspIleIleAlaThrAspIleGlnThrLysGluLeuGlnLysGlnIleThrLysIleGlnAsn
CCACAATTTTAAAAGAAAAGCGGGGCATTGGGGGGTACAGTGCAGCGGGAAAGAATAGTAGACATAATAGCAACAGACATACAAACTAAAGAATTACAAAAACAAATTACAAAAATTCAAAA
                                                                            4400

PheArgValTyrTyrArgAspSerArgAspProLeuTrpLysGlyProAlaLysLeuLeuTrpLysGlyGluGlyAlaValValIleGlnAspAsnSerAspIleLysValValProArg
                                                                                                          ORF Q    CysGlnGlu
TTTTCGGCTTTATTACAGGGACAGCCAGAGATCCACTTTGGAAAGGACCAGCAAAGCTCCTCTGGAAAGGTGAAGGGGCAGTAGTAATACAAGATAATAGTGACATAAAAGTAGTGCCAAG
                                                4500

ArgLysAlaLysIleIleArgAspTyrGlyLysGlnMetAlaGlyAspAspCysValAlaSerArgGlnAspGluAsp  *
GluLysGlnArgSerLeuGlyIleMetGluAsnArgTrpGlnValMetIleValTrpGlnValAspArgMetArgIleArgThrTrpLysSerLeuValLysHisHisMetTyrValSer
AAGAAAAGCAAAGATCATTAGGGATTATGGAAAACAGATGGCAGGTGATGATTGTGTGGCAAGTAGACAGGATGAGGATTAGAACATGGAAAAGTTTAGTAAAACACCATATGTATGTTT
                                                4600

GlyLysAlaArgGlyTrpPheTyrArgHisHisTyrGluSerProHisProArgIleSerSerGluValHisIleProLeuGlyAspAlaArgLeuValIleThrThrTyrTrpGlyLeu
CAGGGAAAGCTAGGGGATCGTTTTTATAGACATCACTATGAAAGCCCTCATCCAAGAATAAGTTCAGAAGTACACATCCCACTAGGGGATGCTAGATTGGTAATAACAACATATTGGGGTC
            4700                                                                                            4800

HisThrGlyGluArgAspTrpHisLeuGlyGlnGlyValSerIleGluTrpArgLysLysArgTyrSerThrGlnValAspProGluLeuAlaAspGlnLeuIleHisLeuTyrTyrPhe
TGCATACAGGAGAAAGAGACTGGCATCTGGGTCAGGGAGTCTCCATAGAATGGAGGAAAAAGAGATATAGCACACAAGTAGACCCTGAACTAGCAGACCAACTAATTCATCTGTATTACT
                                                                                    4900

AspCysPheSerAspSerAlaIleArgLysAlaLeuLeuGlyHisIleValSerProArgCysGluTyrGlnAlaGlyHisAsnLysValGlySerLeuGlnTyrLeuAlaLeuAlaAla
TTGACTGTTTTTCAGACTCTGCTATAAGAAAGGCCTTATTAGGACATATAGTTAGCCCTAGGTGTGAATATCAAGCAGGACATAACAAGGTAGGATCTCTACAATACTTCCCACTACCAC
                                                                            5000

LeuIleThrProLysLysIleLysProProLeuProSerValThrLysLeuThrGluAspArgTrpAsnLysProGlnLysThrLysGlyHisArgGlySerHisThrMetAsnGlyHis
CATTAATAACACCAAAAAAGATAAAGCCACCTTTGCCTAGTGTTACGAAACTGACAGAGGATACATGGAACAAGCCCCAGAAGACCAAGGGCCACACAGCCCACCCACACAATGAATGGAC
      *                                         5100

FIG. 4D

ACTAGAGCTTTTAGAGGAGCTTAAGAATGAAGCTGTTAGACATTTTCCTAGGATTTGGCTCCATCGCTTACGGCAACATATCTATGAAACTTATCGGGATACTTGGGCAGGAGTCGAAGC

CATAATAACAATTCTGCAACAACTGCTGTTTATCCATTTCAGAATTGGGTGTCGACATAGCAGAATAGGCGTTACTCAACAGAGGAGAGCAAGAAATGGAGCCAGTAGATCCTAGACTAG

AGCCCTCGAACCATCCAGGAAGTCAGCCTAAAACTGCTTGTACCACTTGCTATTGTAAAAAGTGTTCCTTTCATTCCCAAGTTTGTTTCACAACAAAGCCTTAGGCATCTCCTATGGCA

GGAAGAAGCCGAGACACCGACGAAGACCTCCTCAAGGCAGTCAGACTCATCAAGTTTCTCTATCAAAGCAGTAAGTAGTACATGTAATGCAACCTATACAAATAGCAATAGCAGCATTAG

                                                                                    ENV ~ LysGluGlnLysThr
TAGTAGCAATAATAATAGCAATAGTTGTGTGTCCATAGTAATCATAGAATATAGGAAAATATTAAGACAAAGAAAAATAGACACGTTAATTGATAGACTAATAGAAAGAGCAGAAGACA

ValAla[Met]ArgValLysGluLysTyrGlnHisLeuTrpArgTrpGlyTrpLysTrpGlyThrMetLeuLeuGlyIleLeuMetIleCysSerAlaThrGluLysLeuTrpValThrVal
GTGGCAATGAGACTGAACGAGAAATATCAGCACTTGTGGAGATGGGGGTGGAAATGGGGCACCATGCTCCTTGGGATATTGATGATCTGTAGTGCTACAGAAAAATTGTGGGTCACAGTC

TyrTyrGlyValProValTrpLysGluAlaThrThrThrLeuPheCysAlaSerAspAlaLysAlaTyrAspThrGluValHisAsnValTrpAlaThrHisAlaCysValProThrAsp
TATTATGGGGTACCTGTGTGGAAGGAAGCAACCACCACTCTATTTTGTGCCATCAGATGCTAAAGCATATGATACAGAGGTACATAATGTTTGGGCCACACATGCCTGTGTACCCACAGAC

ProAsnProGlnGluValValLeuValAsnValThrGluAsnPheAsnMetTrpLysAsnAspMetValGluGlnMetHisGluAspIleIleSerLeuTrpAspGlnSerLeuLysPro
CCCAACCCACAAGAAGTAGTATTGGTAAATGTGACAGAAAATTTTAACATGTGGAAAAATGACATGGTAGAACAGATGCATGAGGATATAATCAGTTTATGGGATCAAAGCCTAAAGCCA

CysValLysLeuThrProLeuCysValSerLeuLysCysThrAspLeuGlyAsnAlaThrAsnThrAsnSerSerAsnThrAsnSerSerSerGlyGluMetMetMetGluLysGlyGlu
TGTGTAAAATTAACCCCACTCTGTGTTAGTTTAAAGTGCACTGATTTGGGCAATGCTACTAATACCAATAGTAGTAATACCAATAGTAGTAGCGGGGAAATGATGATGGAGAAAGGAGAG

IleLysAsnCysSerPheAsnIleSerThrSerIleArgGlyLysValGlnLysGluTyrAlaPhePheTyrLysLeuAspIleIleProIleAspAsnAspThrThrSerTyrThrLeu
ATAAAAAACTGCTCTTTCAATATCAGGACAAGCATAAGAGGTAAGGTGCAGAAAGAATATGCATTTTTTTATAAACTTGATATAATACCAATAGATAATGATAGTACCAGCTATACGTTG

ThrSerCysAsnThrSerValIleThrGlnAlaCysProLysValSerPheGluProIleProIleHisTyrCysAlaProAlaGlyPheAlaIleLeuLysCysAsnAsnLysThrPhe
ACAAGTTGTAACACCTCAGTCATTACACAGGCCCTGTCCAAAGGTATCCTTTGAGCCAATTCCCATACATTATTGTGCCCCGGCTGGTTTTGCGATTCTAAAATGTAATAATAAGACGTTC

AsnGlyThrGlyProCysThrAsnValSerThrValGlnCysThrHisGlyIleArgProValValSerThrGlnLeuLeuLeuAsnGlySerLeuAlaGluGluGluValValIleArg
AATGGAACAGGACCATGTACAAATGTCAGCACAGTACAATGTACACATGGAATTAGCCCAGTAGTATCAACTCAACTGCTGTTGAATGGCAGTCTAGCAGAAGAAGAGGTAGTAATTAGA

FIG. 4E

SerAlaAsnPheThrAspAsnAlaLysThrIleIleValGlnLeuAsnGlnSerValGluIleAsnCysThrArgProAsnAsnAsnThrArgLysSerIleArgIleGlnArgGlyPro
TCTGCCAATTTCACAGACAATGGTAAAACCATAATAGTACAGCTGAACCAATCTGTAGAAATTAATTGTACAAGACCCAACAACAATACAAGAAAAAGTATCCGTATCCAGAGGGGCACCA
6700

GlyArgAlaPheValThrIleGlyLysIleGlyAsnMetArgGlnAlaHisCysAsnIleSerArgAlaLysTrpAsnAlaThrLeuLysGlnIleAlaSerLysLeuArgGluGlnPhe
GGGAGAGCATTTGTTACAATAGGAAAAATAGGAAATATGAGACAAGCACATTGTAACATTAGTAGAGCCAAAATGGAATGCCACTTTAAAACAGATAGCTAGCAAATTAAGAGAACAATTT
6800

GlyAsnAsnLysThrIleIlePheLysGlnSerSerGlyGlyAspProGluIleValThrHisSerPheAsnCysGlyGlyGluPhePheTyrCysAsnSerThrGlnLeuPheAsnSer
GGAAATAATAAAACAATAATCTTTAAGCAATCCTCAGGAGGGGACCCAGAAATTGTAACGCACAGTTTTAATTGTCCAGCGCGAATTTTTCTACTGTAATTCAACACAACTGTTTAATAGT
6900

ThrTrpPheAsnSerThrTrpSerThrGluGlySerAsnAsnThrGluGlySerAspThrIleThrLeuProCysArgIleLysGlnPheIleAsnMetTrpGlnGluValGlyLysAla
ACTTGGTTTAATAGTACTTGGAGTACTGAAGGGTCAAATAACACTGAAGGAAGTGACACAATCACACTCCCATGCAGAATAAAACAATTTATAAACATGTGGCAGGAAGTAGGAAAAGCA
7000

MetTyrAlaProProIleSerGlyGlnIleArgCysSerSerAsnIleThrGlyLeuLeuLeuThrArgAspGlyGlyAsnAsnAsnAsnGlySerGluIlePheArgProGlyGlyGly
ATGTATGCCCCTCCCATCAGCGGACAAATTAGATGTTCATCAAATATTACAGGGGCTGGTATTAACAAGAGATGGTGGTAATAACAACAATGGGTCCGAGATCTTCAGACCTGGAGGAGGA
7100                                                                                                                  7200

AspMetArgAspAsnTrpArgSerGluLeuTyrLysTyrLysValValLysIleGluProLeuGlyValAlaProThrLysAlaLysArgArgValValGlnArgGluLysArgAlaVal
GATATGAGGGACAATTGGAGAAGTGAATTATATAAATATAAAGTAGTAAAAATTGAACCATTAGGAGTAGCACCCACCAACGCCAAAGAGAAGAGTGGTGCAGAGAGAAAAAAGAGCAGTG

GlyIleGlyAlaLeuPheLeuGlyPheLeuGlyAlaAlaGlySerThrMetGlyAlaArgSerMetThrLeuThrValGlnAlaArgGlnLeuLeuSerGlyIleValGlnGlnGlnAsn
GGAATAGGAGCTTTGTTCCTTGGGTTCTTGGGAGCAGCAGGAAGCACTATGGGCGCGACCGTCAATGACGCTGACGGTACAGGCCAGACAATTATTGTCTGGTATAGTGCAGCAGCAGAAC
7300

AsnLeuLeuArgAlaIleGluAlaGlnGlnHisLeuLeuGlnLeuThrValTrpGlyIleLysGluLeuGlnAlaArgIleLeuAlaValGluArgTyrLeuLysAspGlnGlnLeuLeu
AATTTGCTGAGGGCTATTGAGGCCCAACAGCATCTGTTGCAACTCACAGTCTGCCGCATCAAGCAGCTCCAGGCAAGAATCCTGGCTGTGGAAAGATACCTAAAGGATCAACAGCTCCTG
7400

GlyIleTrpGlyCysSerGlyLysLeuIleCysThrThrAlaValProTrpAsnAlaSerTrpSerAsnLysSerLeuGluGlnIleTrpAsnAsnMetThrTrpMetGluTrpAspArg
GGGATTTGGGGTTGCTCTGGAAAACTCATTTGCACCACTGCTGTGCCTTGGAATGCTAGTTGGAGTAATAAATCTCTGGAACAGATTTGGAATAACATGACCTGGATGGAGTGGGACAGA
7500

GluIleAsnAsnTyrThrSerLeuIleHisSerLeuIleGluGluSerGlnAsnGlnGlnGluLysAsnGluGlnGluLeuLeuGluLeuAspLysTrpAlaSerLeuTrpAsnTrpPhe
GAAATTAACAATTACACAAGCTTAATACATTCCTTAATTCAACAATCGCAAAACCAGCAAGAAAAGAATGAACAAGAATTATTGGAATTAGATAAATCGGCAAGTTTGTGGAATTGGTTT
7600

AsnIleThrAsnTrpLeuTrpTyrIleLysIlePheIleMetIleValGlyGlyLeuValGlyLeuArgIleValPheAlaValLeuSerIleValAsnArgValArgGlnGlyTyrSer
AACATAACAAATTGGCTGTGGTATATAAAAATATTCATAATGATAGTAGGAGGCTTGGTAGGTTTAAGAATAGTTTTTGCTGTACTTTCTATAGTGAATAGAGTTAGGCACGGATATTCA
7700                                                                                                                  7800
7900

FIG. 4F

EP 1 231 271 B2

ProLeuSerPheGlnThrHisLeuProThrProArgGlyProAspArgProGluGlyIleGluGluGluGlyGlyGluArgAspArgAspArgSerIleArgLeuValAsnGlySerLeu
CCATTATCGTTTCAGACCCACCTCCCAACCCCGAGGGGACCCGACAGGCCCGAAGGAATAGAAGAAGAACGTGGAGAGAGAGACAGAGACAGATCCATTCGATTAGTGAACGGATCCTTA
                                                                        8000

AlaLeuIleTrpAspAspLeuArgSerLeuCysLeuPheSerTyrHisArgLeuArgAspLeuLeuLeuIleValThrArgIleValGluLeuLeuGlyArgArgGlyTrpGluAlaLeu
GCCACTTATCTGGCACGATCTGCCGGAGCCTGTGCCTCTTCAGCTACCACCGCTTGAGAGACTTACTCTTGATTGTAACGAGGATTGTGGAACTTCTGGCACGCAGGGGGTGCCAAGCCCTC
                                                                        8100

LysTyrTrpTrpAsnLeuLeuGlnTyrTrpSerGlnGluLeuLysAsnSerAlaValSerLeuLeuAsnAlaThrAlaIleAlaValAlaGluGlyThrAspArgValIleGluValVal
AAATATTGGTGGAATCTCCTACAGTATTGGAGTCAGGAACTAAAGAATAGTGCTGTTAGCTTGCTCAATGCCACAGCCATAGCAGTAGCTGAGGGGACAGATAGGGTTATAGAAGTAGTA
                                                                        8200

GlnGlyAlaCysArgAlaIleArgHisIleProArgArgIleArgGlnGlyLeuGluArgIleLeuLeu *
                 ORF F ➤ AspArgAlaTrpLysGlyPheCysTyrLys[Met]GlyGlyLysTrpSerLysSerSerValValGlyTrpProThrVal
CAAGGAGCTTGTAGAGCTATTCGCCCACATACCTAGAAGAATAAGACAGGGCTTGGAAAGGATTTTGCTATAAGATCGGTGGCAAGTGGTCAAAAAGTAGTGTGGTTGGATGGCCTACTGT
        8300                                                            8400

ArgGluArgMetArgArgAlaGluProAlaAlaAspGlyValGlyAlaAlaSerArgAspLeuGluLysHisGlyAlaIleThrSerSerAsnThrAlaAlaThrAsnAlaAlaCysAla
AAGGGAAAGAATGAGACGAGCTGAGCCAGCAGCAGATGGCGTGGGAGCAGCATCTCGAGACCTGGAAAAACATGGAGCAATCACAAGTAGCAATACAGCAGCTACCAATGCTGCTTGTGC
                                                                        8500

TrpLeuGluAlaGlnGluGluGluGluValGlyPheProValThrProGlnValProLeuArgProMetThrTyrLysAlaAlaValAspLeuSerHisPheLeuLysGluLysGlyGly
CTCCCTAGAAGCACAAGAGGAGGAGGACGTCGGTTTTCCAGTCACACCTCAGGTACCTTTAAGACCAATGACTTACAAGGCAGCTGTAGATCTTAGCCACTTTTTAAAAGAAAAGGCGGCG
                                                                        8600

LeuGluGlyLeuIleHisSerGlnArgArgGlnAspIleLeuAspLeuTrpIleTyrHisThrGlnGlyTyrPheProAspTrpGlnAsnTyrThrProGlyProGlyValArgTyrPro
ACTCGAAGGCCTAATTCACTCCCAACGAAGACAAGATATCCTTGATCTGTGGATCTACCACACAAGGCTACTTCCCTGATTGGCAGAACTACACACCAGGGCCAGGGGTCAGATATCC
                                                                        8700

LeuThrPheGlyTrpCysTyrLysLeuValProValGluProAspLysValGluGluAlaAsnLysGlyGluAsnThrSerLeuLeuHisProValSerLeuHisGlyMetAspAspPro
ACTGACCTTTGGATGGTGCTACAAGCTAGTACCAGTTGAGCCAGATAAGGTAGAAGAGGCCAATAAAGGAGAGAACACCAGCTTGTTACACCCTGTGAGCCTGCATGGAATGGATGACCC
                                                                        8800

GluArgGluValLeuGluTrpArgPheAspSerArgLeuAlaPheHisHisValAlaArgGluLeuHisProGluTyrPheLysAsnCys *
TGAGAGAGAAGTGTTAGAGTGCAGGTTTGACAGCCGCCTAGCATTTCATCACGTGGCCCCGAGAGCTGCATCCGGAGTACTTCAAGAACTGCTGACATCGAGCTTGCTACAAGGGACTTTC
        8900                                                            9000

CCCTCGGGACTTTCCAGGGAGGCGTGGCCTCGGCCGGGACTCGGGAGTGCCCGAGCCCTCAGATCCTGCATATAAGCAGCTGCTTTTTGCCTGTACTGGCTCTCTCTCGGTTAGACCAGATTT
                                                                        9100
                                          HindIII.
GAGCCTCGGAGCCTCTCTGGCTAACTAGGGAACCCACTGCTTAAGCCTCAATAAAGCTTGCCTTGAGTCCTTCA
                                                  9193

FIG. 5

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 8324800 A
- FR 8407151
- EP 0178978 A
- GB 8423659 A

### Non-patent literature cited in the description

- **R.C. GALLO et al.** *Science,* 1984, vol. 224, 500
- **M.G. SARNGADHARAN et al.** *Science,* 1984, vol. 224, 506
- **M. JAY LEVY et al.** *Science,* 1984, vol. 225, 840-842
- **Schupbach et al.** *Science,* 04 May 1984, vol. 224, 503-505
- **Sanger et al.** *Proc. Natl. Acad. Sci. USA,* 1977, vol. 74, 5463
- **SCOTTO J. et al.** *Hepatology,* 1983, vol. 3, 379-384
- **Alizon, M. ; Sonigo, P. ; Barré-Sinoussi, F. ; Chermann, J.C. ; Tiollais, P. ; Montagnier, L. ; Wain-Hobson, S.** Molecular cloning of lymphadenopathy-associated virus. *Nature,* 1984
- **Arya, S.K. ; Gallo, R.C. ; Hahn, B.H. ; Shaw, G.M. ; Popovic, M. ; Salahuddin, S.Z. ; Wong-Staal, F.** Homology of genome of AIDS-associated virus with genomes of human T-cell leukemia lymphoma viruses. *Science,* 1984, vol. 225, 927-930
- **Barré-Sinoussi, F. ; Chermann, J.C. ; Rey, F. ; Nugeybe, M.T. ; Chamaret, S. ; Gruest, J. ; Dauguet, C. ; Axler-Blin, C. ; Vézinet-Brun F. ; Rouzioux, C.** Isolation of a T-lymphotropic retrovirus from a patient at risk of Acquired Immune Deficiency Syndrome (AIDS). *Science,* 1983, vol. 220, 868-870
- **Biggen, M.D. ; Gibson, T.J. ; Hong, G.F.** Buffer gradient gels and S label as an aid to rapid DNA sequence determination. *Proc. Natl. Acad. Sci. USA,* 1983, vol. 80, 3963-3965
- **Bird, A.P.** DNA methylation and the frequency of CpG in animal DNA. *Nucl. Acids Res.,* 1980, vol. 8, 1499-1504
- **Brun-Vézinet, F. ; Rouzioux, C. ; Barré-Sinoussi, F. ; Klatzmann, D. ; Saimot, A.G. ; Rozembaum, W. ; Montagnier, L. ; Chermann, J.C.** Detection of IgG antibodies to lymphadenopathy associated virus (LAV) by ELISA, in patients with acquired immuno-deficiency syndrome of lymphadenopathy syndrome. *Lancet,* 1984, vol. I, 1253-1256
- **Chen, H.R. ; Barker, W.C.** Nucleotide sequences of the retroviral long terminal repeats and their adjacent regions. *Nucl. Acids Res.,* 1984, vol. 12, 1767-1778
- **Chen, I.S.Y. ; Mc Laughlin, J. ; Gasson, J.C. ; Clark, S.C. ; Golde, D.W.** Molecular characterization of genome of a novel human T-cell leukaemia virus. *Nature,* 1983, vol. 305, 502-505
- **Chiu, I.M. ; Callahan, R. ; Tronick, S.R. ; Scholm, J. ; Aaronson, S.A.** Major pol gene progenitors in the evolution of oncornaviruses. *Science,* 1984, vol. 223, 364-370
- **Cianciolo, G.J. ; Kipnis, R.J. ; Snyderman, R.** Similarity between p15E of murine and feline viruses and p2! of HTLV. *Nature,* 1984, vol. 311, 515
- **Daly, H.M. ; Scott, G.L.** Fatal AIDS in a haemophiliae in the U.K. *Lancet,* 1983, vol. II, 1190
- **Dittmar, K.J. ; Moelling, K.** Biochemical properties of p15-associated protease in an avion RNA tumor virus. *J. Virol,* 1973, vol. 28, 106-113
- **Donehower, L.A. ; Huang, A.L. ; Hager, G.L.** Regulatory and coding potential of the mouse mammary tumour virus long terminal redundancy. *J. Virol.,* 1981, vol. 37, 226-238
- **Gottlieb, M.S. ; Schroff, R. ; Schanler, H.M. ; Weisman, J.D. ; Fan P.T. ; Wolf R.A. ; Saxon, A.** Pneumocytis carinii pneumonia and mucosal candidiasis in previously healthy homosexual men : Evidence of a new acquired cellular immuno-deficiency. *N.Engl. J. Med.,* 1981, vol. 305, 1426-1431
- **Hahn, B.H. ; Shaw, G.M. ; Arya, S.U. ; Popovic, M. ; Gallo, R.C. ; Wong-Stall, F.** Molecular cloning and characterization of the HTLV-III virus associated with AIDS. *Nature,* 1984, vol. 312, 166-169
- **Harris, J.D. ; Scott, J.V. ; Taylor, B. ; Brahic, M. ; Stowring, L. ; Ventura, P. ; Haase, A.T ; Peluso, R.** Visna virus DNA : discovery of a novel gapped structure. *Virology,* 1981, vol. 113, 573-583

- **Kiyokawa, T. ; Yoshikura, H. ; Hattori, S. ; Secki, M. ; Yoshida, M.** Envelope proteins of human T-cell leukemia virus : expression in Escherischia coli and its- application to studies of env gene functions. *Proc. Natl. Acad. Sci. USA,* 1984, vol. 81, 6202-6206

- **Kiatzmann, D. ; Barré-Sinoussi, F. ; Nugeyre, M.T. ; Dauguet, C. ; Vilmer, E. ; Griscelli, C. ; Brun-Vézinet, F. ; Rouzioux, C. ; Gluckman, J.C. ; Chermann, J.C.** Selective tropism of lymphadenopathy associated virus (LAV) for helper-inducer T-lymphocytes. *Sciences,* 1984, vol. 225, 59-63

- **Kozak, M.** Compilation and analysis of sequences upstream from the transcriptional start site in eucaryotic mRNAs. *Nucl. Acids Res.,* 1984, vol. 12, 857-872

- **Levy, J.A. ; Hoffman, A.D. ; Kramer, S.M. ; Lanois, J.A ; Shimabukuro, J.M ; Oskiro, L.S.** Isolation of lymphocytopathic retroviruses from San Francisco patients with AIDS. *Science,* 1984, vol. 225, 840-842

- **Masur, H. ; Michelis, M.A. ; Greene, J.B. ; Onovato, I. ; Van de Stowe, R.A. ; Holzman, R.S. ; Wormser, G. ; Brettman, L. ; Lange, M. ; Murray, H.W.** An outbreak of community-acquired pneumocystis carinii pneumonia : Initial manifestation of cellular immune dysfunction. *N. Engl. J. Med.,* 1981, vol. 305, 1431-1438

- **Misra, T.K. ; Grandgenett, D.P ; Parsons, J.T.** Avian retrovirus pp32 DNA-binding protein. I. Recognition fo specific sequences on retrovirus DNA terminal repeats. *J. Virol,* 1982, vol. 44, 330-343

- **Montagnier, L. et al.** A new human T-lymphotropic retrovirus : characterization and possible role in lymphadenopathy and acquired immune deficiency syndromes. *In human T-cell leukemia/lymphoma viruses,* 1984, 363-370

- **Oroszlan, S. ; Copeiand, T.D. ; Kalyanaraman, V.S. ; Sarngadharan, M.G ; Schultz, A.M. ; Gallo, R.C.** Chemical analysis of human T-cell leukemia virus structural proteins. *In HTLVS,* 1984, 101-110

- **Piot, P. ; Quinn, T.C. ; Taelmann, H. ; Feinsod, F.M et al.** Acquired immunodeficiency syndrome in a heterosexual population in Zaire. *Lancet,* 1984, vol. II, 65-69

- **Popovic, M. ; Sarngadharan, M.G. ; Read, E ; Gallo, R.C.** Detection, isolation, and continuous production of cytopathic retroviruses (HTLV-III) from patients with AIDS and pre-AIDS. *Science,* 1984, vol. 224, 497-500

- **Querat, G. ; Barban, N. ; Sauze, N. ; Filippi, P. ; Vigne, R. ; Russo, P. ; Vitu, C.** Highly lytic and persistent lentiviruses naturally present in sheep with progressive pneumonia are genetically distinct. *J. Virol,* 1984, vol. 52, 672-679

- **Raba, M. ; Limburg, K. ; Burghagen, M. ; Katze, J.R. ; Simsek, M. ; Heckman, J.E. ; Rajbhandary, U.L. ; Gross, H.J.** Nucleotide sequence fo three isoaccepting lysine tRNAs from rabbit liver and SV40-transformed mouse fibroblasts. *Eur. J. Biochem.,* 1979, vol. 97, 305-318

- **Rice, N.R. ; Stephens, R.M. ; Couez, D. ; Deschamps, J. ; Kettmann, R. ; Burny, A. ; Gilden, R.V.** The nucleotide sequence of the env gene and post-env region of bovine leukemia virus. *Virology,* 1984, vol. 138, 32-93

- **Sagata, N. ; Yasunaga, T. ; Ogawa, Y. ; Tsuzuku-Kawamura, J. ; Ikawa, Y.** Bovine leukemia virus : Unique structural features of its long terminal repeats and its evolutionary relationship to human T-cell leukemia virus. *Proc. Natl. Acad. Sci. USA,* 1984, vol. 81, 4741-4745

- **Sanger, F. ; Nicklen, S. ; Coulsen, A.R.** DNA sequencing with chain terminating inhibitors. *Proc. Natl. Acad. Sci. USA,* 1977, vol. 74, 5463-5467

- **Schwartz, D.E. ; Tizard, R. ; Gilbert, W.** Nucleotide sequence of Rous sarcoma virus. *Cell,* 1983, vol. 32, 853-869

- **Schüpbach, J. ; Popovic, M. ; Gilden, R.V. ; Gonda, M.A. ; Sarngadharan, M.G. ; Gallo, R.C.** Serological analysis of a subgroup of human T-lymphotropic retroviruses (HTLV-III) associated with AIDS. *Science,* 1984, vol. 224, 503-505

- **Seiki, M. ; Hattori, S. ; Hirayama, Y ; Yoshida, M.** Human adult T-cell leukemia virus : complete nucleotide sequence of the provirus genome integrated in leukemia cell DNA. *Proc. Natl. Acad. Sci. USA,* 1983, vol. 80, 3618-3622

- **Shaw, G.M. ; Hahn, B.H. ; Arya, S.K. ; Groopman, J.E. ; Gallo, R.C. ; Wong-Staal, F.** Molecular characterization of human T-cell leukemia (lymphotropic) virus type III in the Acquired Immune Deficiency Syndrome. *Science,* 1984, vol. 226, 1165-1171

- **Shimotohno, k. ; Temin, H.M.** Spontaneous variation and synthesis in the U3 region of the long terminal repeat of avion retroviruses. *J. Virol,* 1982, vol. 41, 1636171

- **Shimotohno, K. ; Golde, D.M. ; Miwa, M. ; Sugimura, T. ; Chen, I.S.Y.** Nucleotide sequence analysis fo the long terminal repeat of human T-cell leukemia virus type II. *Proc. Natl. Acad. Sci. USA,* 1984, vol. 31, 1079-1083

- **Shinnick, T.M. ; Lerner, R.A. ; Sutcliffe, J.G.** Nucleotide sequence of Moloney murine leukemia viruse. *Nature,* 1981, vol. 293, 543-548

- **Srinivasan, A. ; Reddy, E.P. ; Dunn, C.Y. ; Aaronson, S.A.** Molecular dissection of transcriptional control elements with the long terminal repeat of retrovirus. *Science,* 1984, vol. 223, 286-289

- **Staden, R.** Automation of the computer handling of gel reading data produced by the shotgun method of DNA sequencing. *Nucl. Acids. Res,* 1982, vol. 10, 4731-4751

- **Temin, H.** Structure, variation and synthesis of retrovirus long terminal repeat. *Cell,* 1981, vol. 27, 1-3

- **Weinberg, R.A.** Fewer and fewer oncogenes. *Cell,* 1982, vol. 30, 3-9